# EUROPEAN PATENT SPECIFICATION

(11) **EP 3 172 318 B1**
(45) Date of publication and mention of the grant of the patent: **31.03.2021**
(21) Application number: 15763497.3
(22) Date of filing: 20.07.2015
(51) Int. Cl.: C12N 7/01, A61K 39/12, C12N 7/00, C12N 7/08, A61K 39/00

(54) **ATTENUATED STRAIN OF PRRS AND POTENTIAL USE IN IMMUNISING PREPARATIONS**
ABGESCHWÄCHTER STAMM VON PRRS UND POTENTIELLE VERWENDUNG IN IMMUNISIERENDEN PRÄPARATEN
SOUCHE ATTÉNUÉE DU VSRRP ET SON UTILISATION POTENTIELLE DANS DES PRÉPARATIONS D'IMMUNISATION

(30) Priority: 21.07.2014 IT RM20140407
(43) Date of publication of application: 31.05.2017
(73) Proprietor: FATRO S.p.A., 40064 Ozzano dell'Emilia (BO) (IT)
(72) Inventor: FERRARI, Maura, 25124 Brescia (BS) (IT); ALBORALI, Giovanni, Loris, 25124 Brescia (BS) (IT); LOMBARDI, Guerino, 25124 Brescia (BS) (IT); PONGOLINI, Stefano, 25124 Brescia (BS) (IT)
(74) Representative: Capasso, Olga
(86) International application number: PCT/EP2015/066561
(87) International publication number: WO 2016/012406

(56) References cited:
- EP-A1- 2 712 927
- WO-A1-98/00165
- WO-A1-2013/017568
- US-A1- 2012 189 655
- J. HU ET AL: "Porcine Reproductive and Respiratory Syndrome Virus Vaccines: Current Status and Strategies to a Universal Vaccine", TRANSBOUNDARY AND EMERGING DISEASES, vol. 61, no. 2, 24 January 2013 (2013-01-24), pages 109-120, XP055174774, ISSN: 1865-1674, DOI: 10.1111/tbed.12016
- DATABASE EMBL [Online] 13 February 2011 (2011-02-13), "Porcine reproductive and respiratory syndrome virus strain Cresa3262, partial genome.", XP002751079, retrieved from EBI accession no. EM_STD:JF276431 Database accession no. JF276431
- DATABASE Geneseq [Online] 12 December 2002 (2002-12-12), "PRRS virus ORF2 DNA sequence #2.", XP002751080, retrieved from EBI accession no. GSN:ABS76689 Database accession no. ABS76689
- DATABASE Geneseq [Online] 8 May 2002 (2002-05-08), "Canarypox virus vector pJP115 DNA encoding PRRSV ORF2.", XP002751081, retrieved from EBI accession no. GSN:ABK15632 Database accession no. ABK15632
- DATABASE EMBL [Online] 20 May 2008 (2008-05-20), "Porcine reproductive and respiratory syndrome virus (prrsv) recombinant poxvirus vaccine.", XP002751082, retrieved from EBI accession no. EM_PAT:DJ388904 Database accession no. DJ388904
- DATABASE EMBL [Online] 1 November 2004 (2004-11-01), "Sequence 1 from Patent EP1469076.", XP002751083, retrieved from EBI accession no. EM_PAT:CQ891690 Database accession no. CQ891690
- DATABASE EMBL [Online] 24 September 2013 (2013-09-24), "Porcine reproductive and respiratory syndrome virus isolate 3027 GP5 gene, complete cds.", XP002751084, retrieved from EBI accession no. EM_NEW:KF181382 Database accession no. KF181382
- DATABASE EMBL [Online] 26 June 2012 (2012-06-26), "Porcine reproductive and respiratory syndrome virus strain Stendal_V852 membrane protein M gene, complete cds.", XP002751085, retrieved from EBI accession no. EM_STD:JN651712 Database accession no. JN651712
- DATABASE EMBL [Online] 20 May 2008 (2008-05-20), "Porcine reproductive and respiratory syndrome virus (prrsv) recombinant poxvirus vaccine.", XP002751086, retrieved from EBI accession no. EM_PAT:DJ388925 Database accession no. DJ388925
- DATABASE EMBL [Online] 24 September 2013 (2013-09-24), "Porcine reproductive and respiratory syndrome virus isolate 1536-23 nucleocapsid gene, complete cds.", XP002751087, retrieved from EBI accession no. EM_STD:KF181340 Database accession no. KF181340

### SEQUENCE LISTING

<110> Istituto Zooprofilattico Sperimentale della Lombardia e dell'Emilia Romagna "Bruno Ubertini" (IZSLER)
<120> ATTENUATED STRAIN OF PRRS AND POTENTIAL USE IN IMMUNISING PREPARATIONS
<130> 126779 PCT
<150> RM2014A000407
   <151> 2014-07-21
<160> 21
<170> PatentIn version 3.5
<210> 1
   <211> 15012
   <212> DNA
   <213> Porcine reproductive and respiratory syndrome virus
<400> 1
   tgtgtagggt attcccccta cgcgcgcaac acttctagtg tttgtgtgcc ttggaggcgt       60
   gggtatagcc ccgccccacc tcttggcccc tgttctagcc caacaggaat ccttctccct      120
   cggggcgagt gcgccgcctg ctgctccctt gcagtaggaa ggacctcccg agtatttccg      180
   gagagcacct gctttacggg acctccaccc tttaaccatg tctgggacgt tttcccggtg      240
   catgtgcacc ccggctgccc gggtattttg gaacgccggc caagtctttt gcacacggtg      300
   tctcagtgca cggcctcttc tccctccgga gcttcaggac actgatcttg gtggaattgg      360
   cttattttac aggcccaaag acaagcttcc ctggaaagtt cccattggca tcccccaagt      420
   ggagtgcacg ccatccgggt gctgctggct gtcagccgtt ttccctttgg cgcgcatgac      480
   ctccggcaac cacaatttcg tccaacgact tgtgaaagtt gctgatgtgt tgtaccgtga      540
   cggttgcttg acccctcgac accttcgtga actccaagtt tacgagcgtg gttgcaactg      600
   gtacccgatc acggggcccg tgcccgggat gggtttgttc gcgaactcca tgcacgtgtc      660
   tgaccaaccg ttccctggtg ccacccatgt gttgacgaac tcgcctctgc ctcagcaggc      720
   ttgtcgacag ccgttctgcc catttgagga ggcccattct gacgtgtata agtggaagga      780
   agttgtggtt ttcatggact cctcccccaa cggtcgatct cgcatgatgt ggatgccgga      840
   gtccggtgat tcagctgccc tggaggcatt accgcctgag ttagaacgtc aagtcgaaat      900
   cctcattcgg agctttcctg cccaccatct tgttgacctt accgactggg agctcactga      960
   gtcccctgag cacggttttt ccttcggcac gtcttatcct tgtggttacc ttgcccaaaa     1020
   cccttatggt tttgatggca agtgttggct ctcctgcttc ttgggcctgc cgaccaaagt     1080
   ttggcgtcat gaggagtatc tagctagcgc tttcggttat cagaccaagt ggggcgtaca     1140
   tggcaaatac ctccagcgta ggcttcaaat taacggtgtc cgtgctgcgg tcgatcctga     1200
   cggtcccatc cacgttgaag cgctgtcttg cccccagtct tggattaggc acctgacttt 1260
   ggacgatgat gttaccccag gattcgttcg cctgatgtcc ctccgcattg taccgaacac 1320
   agagcctact accctcccaa tcttccggtt tggggcgcat aaatggtatg gggcagctgg 1380
   caaacgagct cgcgccaagc gtgctgccaa aggtaagggg gattcgaatt ccacccccga 1440
   agttgcccga gtggctccca ctcacggggt tatcacctat tccccaccgg cagatgggtc 1500
   ctgtggttgg catgttattg ccgccgtgat gaaccatatg atgaatggtg aactcacgtc 1560
   ccctctgact ccgtacaaca gaccagagga cgactgggct tctgattatg atcttgtcaa 1620
   gacaatccaa tatttgcaac tgcccgcaac cgtggttcgg gcccgcgcct gtcccaacgc 1680
   taagtacctt gttaagctta atggggtcca ttgggaagta gagatgaggc cgggagtggc 1740
   ccctcgctcc cttcctcgcg aatgtgtagt tggcgtctgc tctgaaggct gcgtcacgcc 1800
   gcctctttca gagagggtgt tgcctgaccg tgcactcgag gccctggcat ctgcttacag 1860
   attgccttcc gactgtgtct gtgacgctgt tgctgacttc ctctccaacc cgccctctca 1920
   aggatcctgg actctcgaca gaatgctaac ctccccgtca ccagaacagt ctggtttttc 1980
   tagcttgtac agactgctat tggaggttgt tccgcagaaa tgcggagcca cagagggggc 2040
   tttcgtctat gccgttgaca ggatgttgaa ggattgtccg agccctaaac aagccatggc 2100
   ccttttgggg aaaatcaaaa tcccatcctc gaaggcctcg tctgtgtcct tggacgagtg 2160
   ctttcctaca gatgttttga ctgtccccgg gtcggtgttt caggaagagc cccaaagctt 2220
   tgacaacgcc atcgccctgt gttcgctgga aaaacaaaag gaaactgagg gggcaagccc 2280
   aggaaaagct caagaaagtg attgtaagac cttccgctct gtggttcctg ccgagaaacc 2340
   taacatgcag caagtgccgt tggttgagga tgagcaattg aagctcggcg gttgtgactc 2400
   agccgttgcg acggttgata gtgatcgggg aaatgaaccg ttagacctat cccgatcagc 2460
   cccaactgca acaacaacct ccgtcggagg gcgagcaccc gacaacccgg gccccaacac 2520
   tagcgtccgc tccactaccg ttcaagagtc ggttccgggg aggcccgtgc cccgccttgt 2580
   tgagcgatgc ggcgtggagt cggacaacag caattcgccc ctggatttgt ctaaggcgca 2640
   gaccccagac cggcccctgg acctatcttt agccgcttgg ccagtgaggg ctactgcgtc 2700
   tgaccccggt tgggtcgacg gtaggcgcga acctgtcttc gttaagcctc gcggtgcctt 2760
   ctctgacagc gagtcagtca ttcggtttgg gggggtttct gagactagct ctatcattga 2820
   gtttgaccgg acagaagaaa ctccggtgac tgacgtcccc atcgacttaa caacttcaaa 2880
   cgagactctt tccggggcag accccttcga acttgctgaa cctaagcgtc cgcgcctctc 2940
   cgctcaagct ttgattgacc gaggtggccc acttgctgat gtccatgcaa aaataaaaaa 3000
   tcgagtgtat gaacagtgcc tccaggcttg cgaacccggc agtcgtgcaa ccccagccac 3060
   aaaggaatgg ctcgacaaaa tgtgggagag ggtcgatatg aagacctggc gttgtacctc 3120
   gcagttccaa gcagcctaca tactcgggcc cctcaaattc cttccagata tgattcgtga 3180
   cgcgccacct cctgtcccca ggaaggatcg ggcttgtgac agtgccagct tgaagcaatt 3240
   agtggcacgg tgggagaaga gattgaatgc agtccctctt caaggaccgg ttgagccgga 3300
   gcttggccgg accgctccct cgtccgtgaa tgcccagcca gaagaagcca tcccttccga 3360
   tggaccacct caagcgccgg accctcctgg accaacaagt gctggtaggg gttttaaagg 3420
   acttatatcc cccggcatcc gccttatagg gtccgctagc cagcgcttca tgacatgggt 3480
   ttttgaaatt tactcccatc tcccagcttt tatgctcaca cttttctcgc cacggggctc 3540
   tatggttaca agtgattggc tgtttgcagg tgttgtttta tttgctctcc tgttttgtcg 3600
   ttcttgcccg atactcgggt gcctaccgct attgggtgtc ttttctggtt ctgtgcggtg 3660
   tgttcgtctg ggcgtttttg gctcttggat ggcttttgct gtatttttat tctcgactcc 3720
   aaccaatcca atcggttctt cttgtgacca cgattcgccg gagtgtcatg ctgagctttt 3780
   ggctcttgag cagcgccaac tttgggaacc tgtgcgcagc cttgtggttg gcccctcagg 3840
   cctctcatgc gtcattctgg gtaagctgct cggtgggtca cgttatctct ggcatgttgt 3900
   cttacgttta tgcatgctcg cggacttggc cctttctctt atttatgtgg tgtcccaagg 3960
   gcgttgttac aagtgttggg gaaagtgtat aaggacagct cctgcagagg tggccctcaa 4020
   tgtgtttccc ttctcgcgcg ccactcgttc ttctcttgta tccttgtgcg atcggttcca 4080
   ggcgccaaaa gaggttgacc ccgtgcactt ggcaacaggt tggcgcgggt gttggcgtgg 4140
   tgagagtccc attcatcaag cgcaccagaa gcctatagct tacgccagcc tggatgaaaa 4200
   gaagatatct gcccagacgg tggttgctgt gccgtacgat cctagtcagg ccgtcaaatg 4260
   cttaaaggtt ttgcaggccg gtggagccat cgtggatcaa cctgttcctg aggtcatccg 4320
   tgtgtccgaa gttcctttct cagccccatt ctttccaaag gttccagtca acccagattg 4380
   tagggttgtg gtggattcgg acacctttgt ggccgcggtt cgctgcggtt actcgacggc 4440
   acaactgatc ctaggtcgag gcaactttgc caagttaaac cagaccccac ccaagaactc 4500
   catccccacc aagtcgaccg gtggggcctc ttacaccctt gccgtgaccc aagtgtctgt 4560
   gtggactttg gtacacttca tcatcggcct ttggttgacg tcgcctcagg tgtgtggtcg 4620
   gggaacctca gacccgtggt gttcaaatcc tttttcgtac cctacttatg gccctggagt 4680
   tgtgtgctcc tctcgacttt gtgtgtctgc tgacggggtc accctaccgt tgttttcagc 4740
   cgtggctcaa ctctctggca gagaggtggg ggtttttatt ttggtgctcg tctccttgat 4800
   ctccttagcc catcgcctgg ctctcaaggc agatatgctg gtggtctttt cggcattttg 4860
   tgcttacgcc tggcccatga gctcctggtt gatttgcttc tttcccatac tcctgaggtg 4920
   ggtcaccctt caccctctca ctatgctttg ggtacattca tttctagtgt tttgcctgcc 4980
   agctgccggc gtcctttcac tgggtatatc tggtttcctc tgggcagttg gccgtttcac 5040
   tcaggttgcc ggaattataa caccttatga catccaccaa tatacttccg ggccgcgcgg 5100
   cgcggccgct gtagcaacgg cccctgaagg cacctacatg gccgccgttc ggagagctgc 5160
   cttgaccggt cggactttga ttttcacgcc atcagcagtt ggatccctcc ttgaaggtgc 5220
   ttttaggact aataagccct gcctcaacac ggtgaatgtc gtaggttcct ctctcggctc 5280
   cgggggtgtc ttcaccgtgg gtggcagtaa gataattatc acagccaccc acgtgctgaa 5340
   cggtgatgca gctagggtca ccggcgactc ttacaatcgt atgcacactt ttaaaaccaa 5400
   tggtgattat gcctggtccc gtgctgatga ctggcagggt gtcgctcctg ctgttaaagt 5460
   cgcgaagagg taccgtggcc gtgcctactg gcaaacatca actggtgtcg agcccggcat 5520
   cattggggag gggttcgcct tctgtttcac caactgtggc gactcaggat cacctgtcat 5580
   ttcagagact ggtgacctca tcggggttca caccggctca aacaaacttg gttctggtct 5640
   tgtaacgacc cctgaagggg agacctgctc catcaaagaa actaagctct ctgaactttc 5700
   tagatacttt gcaggtccga gtgtccctct cggagacgtg aggttaagtc cggccattat 5760
   ccctgatgtg gcgtttattc cgagtgattt agcatcgctc cttgcttctg tccctgtaat 5820
   ggagggcggc ctctcaaccg tccaacttct atgtgttttc tttcttctct ggcgcatgat 5880
   gggccatgct tggacaccca ttattgccgt aagcttcttt ttactgaatg aaattcttcc 5940
   agcagttttg gtccgtgctg tattttcttt tgcacttttt gtgcttgcgt gggccacccc 6000
   ttggtctgca caggtgttga tgataagact cctcactgcg gccctcaacc gtaacaggct 6060
   ttctctagct ttttatgcac tcgggggtgt tgtcggctta gctgctgaga tcgggacttt 6120
   tgctggtaaa ctgactgaat tgtcccaggt cttgtccaca tactgctttc tacccagggt 6180
   cattgccatg actagttgtg tccccgtcat catcatcggt gggcttcacg ccctcggtgt 6240
   aatcctatgg ctgttcaagc accgttgcct ccacaatatg ctggttggtg acgggagttt 6300
   ctcaagcgcc ttcttcctgc ggtattttgc agaaggtaat cttaggaaag gcgtctcgca 6360
   gtcttgtggc atgagtaacg aatccttaac ggctgctctg gcctgtaagt tgtcgcaggc 6420
   tgaccttgat tttctatccg gcttgacgaa cttcaagtgc ttcgtgtccg cctcaaacat 6480
   gaaaaatgct gctggccagt acatcgaagc agcgtacgcc aaagccctgc gtcaggagtt 6540
   ggcctctcta gtccaagtcg acaaaatgaa aggggttctg tccaagctcg aagcttttgc 6600
   tgagatggct actccatccc tggatacagg agatgtggtt gttctactcg ggcaacaccc 6660
   tcacgggtcc attcttgaca tcaatgttgg gactgaaagg aagaccgtgt ctgtgcaaga 6720
   aacccgaagc ctaggcgggt ccaaattcag tgtctgcacc gttgtgtcca acacgcccgt 6780
   tgacgccctg accggcattc cacttcagac accgacccca ctctttgaga atggcccacg 6840
   ccaccgcagc gaggatgatg atcttaaggt tgagaggatg aagaaacact gcgtctccct 6900
   cggcttccat aatatcaatg gcaagattta ctgcaaggtc tgggacaagt caaccggtga 6960
   caccttctac acggatgatt cccggtatac ccaagaccat gcctttcagg ataggtcagc 7020
   tgattacaga gatagggact atgagggtgt gcaaatcgcc cctcaacaag gattcgaccc 7080
   gatgtctgaa acccccgttg gcactgttgt aattggcggt atcacgtaca gtaggtacct 7140
   agttaaaggc aaggaggtgt tgatccccaa acccgacaac cattttgaag ccgccaagct 7200
   ttccctcgag caagccctcg ctgggatggg ccagacttgt gaccttacag ctgccgaggt 7260
   ggagaagctg aagcgcatta tcagtcaact ccaaggccta accactgaac aggctttaaa 7320
   ctgttagccg ccagcggttt gacccgctgt ggccgcggcg gcttggttgt gacagaaacg 7380
   gcggtaaaaa ttgtgaaata ccacagtaga actttcactt taggccctct agacttaaaa 7440
   gtcacttctg aggcggaagt aaagaaatca actgagcagg gccacgctgt tgtagcaaac 7500
   ttatgctccg gtgtcgtctt aatgaggcct cacccgccat ctcttgttga tgttcttctg 7560
   atacctggac ttgacacagc acccggcatt caaccagggc atggggccgg aaacatgggt 7620
   gtgaacggtg ccatctggga tttcgaaact gcacccacta aggcagaact cgagttgtcc 7680
   aagcaaataa ttcaggcctg tgaggttagg cgcggggacg ccccgaacct ccaactcccc 7740
   tacaagctcc accctgttag gggagaccct gaacggcgca atggccgtct cattaacacc 7800
   aggttcggag atttgcccta caagaccccc caggacacta ggtccgcgat tcacgcggct 7860
   tgctgcctgc atcccaacgg ggtcccggtg tctgatggca aatctacatt aggcaccact 7920
   ctccagcgtg gttttgagct ttacgttccc acagtacctt acagtgtctt agagtacctt 7980
   gattcacgcc ctgatacccc cttcatgtgt actaaacatg gtacttctga ggctgctgca 8040
   gaggacctcc aaaaatacaa cctgtccact caagggtttg tcctgcctgg agtcctccgc 8100
   ctagttcgta gattcatctt tggccatatt ggaaaggcgc cgccattgta cctcccatca 8160
   acttatcctg ccaaaaactc tatggcagga atcaatggcc aaaggttccc aacaaaggat 8220
   gtccagagca tacctgaggt tgatgaaatg tgcgctcgcg ccgtcaaaga taattggcag 8280
   actacgacgc cttgcaccct caagaaacag tactgttcca aacccaaaac taggactatc 8340
   ctgggcacta ataacttcat tgccttggcc catagatcgg cactcagtgg tgtcacccag 8400
   gcgttcatga agaaagcttg gaaatcccca attgccttgg ggaagaacaa attcaaggag 8460
   ttgcactgcg ctgtcgctgg caggtgcctc gaggccgacc tggcctcctg tgatcgcagt 8520
   accccagcca ttgtgaggtg gtttactgcc aatctcctgt atgaacttgc aggatgtgag 8580
   gaatacttgc ctagctatgt gctcaactgc tgtcacgacc tcgtggcgac tcaggatggc 8640
   gcttttacaa aacgcggtgg tctgtcgtct ggggaccccg tcaccagtgt gtctaacacc 8700
   gtgtattcac tggtgattta tgcccagcac atggtgctgt cagccttgaa aatgggccac 8760
   gaaattggcc ttaaattcct cgaggaacag cttagattcg aggacctcat tgaaatccag 8820
   cccatgttgg tgtactccga tgaccttgtc ttgtatgctg agaagcccac ctttcccaat 8880
   taccattggt gggtcgagca ccttgatttg atgctgggct tcaaaacgga cccgaaaaag 8940
   accatcataa ctgataaacc cagcttcctc ggctgcagaa ttgaggcagg gcggcagtta 9000
   gtccccaatc gcgaccgtat cctggccgct cttgcatacc acatgaaagc gcagaacgcc 9060
   tcagagtatt acgcgtctgc tgccgcaatc ctcatggatt catgtgcttg cattgactat 9120
   gaccctgagt ggtatgagga tctcatctgt ggtattgctc ggtgtgctcg tcaagatggc 9180
   tatagttttc caggcccggc atttttcatg tccatgtggg agaagctgaa aagccacaat 9240
   gaggggaaaa aattccgcca ctgcggcatc tgtgatgcta aggccgacca tgcgtccgcc 9300
   tgtgggcttg acttgtgttt gttccactcg cattttcatc aacactgtcc cgttacactg 9360
   agctgcggtc accatgctgg ttcaaaagag tgcccgcagt gtcagtcacc tgtcggggct 9420
   ggcaagtccc ctcttgacac tgtgttgcag caaatcccgt ataaaccacc tcgcactgtc 9480
   ataatgaagg tgaacaataa aacaacagcc cttgacccgg ggaggtacca gtcccgtcgg 9540
   ggtcttgtcg cagtcaagag gggaattgca ggcaatgagg ttgatcttgc tgacggggac 9600
   taccaggtgg tgcccctcct gccgacctgc aaagatataa acatggtaaa ggtggcttgc 9660
   aatgtgttac tcagcaagtt catagtgggg ccgcccggct ccggaaaaac cacctggtta 9720
   ctgaaccaag ttcaagagga tgttgtcatc tacacaccca cccatcaaac tatgttcgac 9780
   atagtcagtg ccctcaaggt ttgcaggtat tcaatcccag gagcctcagg gctccccttc 9840
   ccaccgcctg ccagatctgg accgtgggtc agacttgtcg ccagcgggca cattcctggc 9900
   cgagtttcat accttgacga ggctgggtat tgcaaccatc tggacatcct cagactgctt 9960
   tccaaaacac cccttgtgtg tttgggtgac cttcaacaac tccaccctgt tggctttgat 10020
   tcctgctgtt atgtttttaa tcagatgcct cacaaacagc tgactaccat ttacaggttc 10080
   ggccccaaca tctgtgctgc tatccagcct tgttacaagg agaaacttga atccaaggca 10140
   aggaacacca gggtagtttt caccattcga cctgtggctt atggtcaagt gctgacacca 10200
   tttcacaagg atcgcgttgg ctcagccata accatagatt catctcaggg ggctaccttt 10260
   gatgttgtga cgctgcattt gccaacacca agatccctga ataaatcccg agcacttgta 10320
   gccattactc gggcgaggca cgggttgttc atctacgatc cccacaacca actccaggag 10380
   ttcttcaacc taacccctga gcgtactgat tgcaatctcg tgttcaaccg cgggggtgaa 10440
   ctggtcgttc tgaattcaga taatgcagtc acaactgtag cgaaggtcct ggaggctggc 10500
   ccgtctcaat ttcgggtatc ggacccgagg tgcaagtccc tcttggccgc ttgctcagtc 10560
   agcctagaag ggagctgcat gccactaccg caagtggctc ataacttggg gttttacttt 10620
   tccccagaca gtccggcgtt cgcacctctg ccaaaagaat tggcaccgca ttggccagtg 10680
   gtcacctgcc agaacaacca agcatggcct gaccgactcg ttgccagcat gcgcccgatt 10740
   gatgcccgtt acagcaagcc tatggttggt gcgggatacg ttgttggacc gtcaaccttt 10800
   ctgggaactc ctggtgtggt gtcatactat ctcacactgt acattaaggg tgaaccccgg 10860
   gccttgccag aaacactcgt ctcaacaggg cgcatagcca cagactgtcg ggagtacctt 10920
   gatgcggcag aagaggaagc agccaaggaa ctcccccatg ctttcatagg tgatgtcaag 10980
   ggtaccacag tgggggggtg tcatcacatc acatcaaagt atctacccag gtccctgccc 11040
   aaggactctg ttgccgtggt tggggtaagt tcgcccggca aggctgctaa agccgtgtgc 11100
   actctcactg atgtgtatct ccccgacctt cggccatatc tgcaaccaga gacagcatca 11160
   aaatgctgga aactcaaatt ggacttcagg gacgtcagac tgatggtttg gaaaggggcc 11220
   accgcctatt ttcagttgga agggcttaca tggtcggcac tacccgacta tgccaggttc 11280
   attcagctgc ccaaagatgc tgttgtgtat attgacccgt gcataggacc ggcaacagcc 11340
   aaccgcaagg tagtgcgaac cactgactgg cgagccgacc tggcagtaac gccatatgat 11400
   tacggtgccc aaactatcct gacaacagct tggttcgagg acctcggacc gcaatggaaa 11460
   atcctggggc tgcagccctt caagcgggca tttggcttcg aaaacactga ggactgggca 11520
   atcctcgcgc gccgtatgaa tgacggcagg gattacaccg attataactg gacttgcgtc 11580
   cgagaacgtc cgcacgctat ctacgggcgc gcccgtgacc acacttatca tttcgcccct 11640
   ggtgcagaat tgcaggtaga gctgggcaaa ccccggctgc cgcctgagtg agtacagcgg 11700
   ccctaaagca atgcaatggg gtcattgtgg agtaaaatca cccagctgtt tgtggatgcc 11760
   tttactgagt tccttatcag tgtggttgac attatcatct ttcttgccat actgttcggg 11820
   ttcaccgtcg caggatggtt actggtcttt ctactcagag tggtttgctc cgcgattctc 11880
   cgttcgcgca ctgccgttca ctctcccgaa ttatcgaagg tcctatgaag gcttgctacc 11940
   caattgcagg ccggatgtcc cacaattcgc aatcaaacac ccattgggca tactttggca 12000
   tatgcgagtt tctcatctaa ttgatgagat ggtttctcgt cgtgtctacc aaaccatgga 12060
   gcattctggc caagcggcct ggaagcaggt ggttgctgaa gccactctca caaagctatc 12120
   gcagcttgac atagtcaccc acttccaaca tttagccgca gtggaggcgg attcttgccg 12180
   cttcctcagc tcgcgactcg tgatgctgaa aaatcttgct gttggcaatg tgagtttgct 12240
   gtacaacacc acgctggacc gtgttgaact catcttccct acaccaggta cgaggcccaa 12300
   actgaccgat ttcagacaat ggctcatcag tgtgcacgcc tccatcttct cttctgtggc 12360
   ctcgtcagtc actttgttcg tggtgctttg gcttcgggtc ccaattctac gctatgtttt 12420
   tggtttccat tggcccacgg caacacgtcg tttgagttaa ctattaatta tactgtatgc 12480
   aagccctgcc tcacccgcga tgcggctcga cagaggctcg aacccggtcg caacatgtgg 12540
   tgcaagatag ggcatgatag gtgtgaggac cgtgaccatg atgagttgtc aatgaccatc 12600
   ccacccgggt acgacaacct caaacttgag ggctattatg cttggctcgc tttcctgtcc 12660
   ttttcctatg cagcccaatt ccatccggag ctgttcggga tagggaatgt gtcacgcgtc 12720
   ttcgtggata aggtgcacca gtttgtttgt gccgtgcatg atggacagaa ttcaaccgtg 12780
   tccaacgggc acaacatttc cgctttgtat gcggcatact atcatcacca aatagacggg 12840
   ggcaattggt ttcatctaga atggttgcgg ccattctttt cctcctggct ggtgctcaac 12900
   atttcatggt ttctgaggcg ttcgcctgca agccctgttt ctcgacacat ctatcagata 12960
   ttaagaccaa cacgacagcg gctgccggtt tcatggtcct tcaagacatc agctgcctcc 13020
   agcctcatgg gggttcagaa acgcaaggca ccagcattca tgggaagcca tctcaatgtc 13080
   gtgtagctgt tggcatcccc cagtacatca cggtaactgc gaatgtgact gatgaatcat 13140
   atttgtacaa tgctgacttg ctaatgcttt ctgcctgctt gttctacgcc tcagaaatga 13200
   gcgaaaaagg cttcaaagtc atctttggga atgtctctgg cgttgtctct gcttgcgtta 13260
   atttcacaga ttatgtggcc catgtgactc aacataccca acagcatcat ctggtaattg 13320
   atcacgttcg gttgctgcac tttttgacac catccacgat gaggtgggct acaaccatcg 13380
   cctgcttgtt cgccattctc ctggcgatat gagatgttct cacaaattgg ggtgtatctt 13440
   gactccttat ccttgttgtt gctggctttt tttgttgtgt accggcttgt cttggtcctt 13500
   tgccgatgga aacggcaaca gctcgacata ccaatacata tataacttga cgatatgcga 13560
   gctgaatggg accgcctggc tgtccaatca ctttcaatgg gcagtcgaaa cctttgtgct 13620
   ttacccggtg gtcactcata tcctctcact gggtttcctc acaacaagtc atttctttga 13680
   cacgctcggc cttggagccg tgtccgtcac aggcttttgt cacacgcggt atgtgctcag 13740
   cagtgtgtac ggcgtttgtg cattagcggc gttcatatgt tttaccatcc gtgccgctaa 13800
   aaattgcatg gcatgccgct atgcccgcac ccggttcacc aatttcattg tggatgaccg 13860
   ggggaggatc catcgatgga aatctccaat agttgtggag aagctgggca aagccgaaat 13920
   cggcaatgat ctcatcacca tcaaacatgt tgtcctcgaa ggggtcaaag ctcaaccctt 13980
   gacaaggacc tcggctgagc aatgggaggc atagatgatt tttgctatga ccctaccgcc 14040
   acacagaaac ttgtgttagc tttcagcatc acatatacac ccatcatgat atacgccctt 14100
   aaggtttcac gcggccgact tctggggctg ttgcacatcc tgatcttttt gaactgctcc 14160
   ttcacgttcg ggtacatgac gtatgtgcat tttcagtcca ccaaccgcgt tgcacttacc 14220
   atgggggccg ttgtcgccct cttgtggggc atttatagtt tcatagagtc atggaagttt 14280
   atcacttcca gatgcagatt gtgttgccta ggccggcgat acattctggc ccctgcccac 14340
   cacgtagaaa gtgctgcagg actccattca atcccagcgt ctggtaaccg agcatacgct 14400
   gtgagaaagc ccggactaac ctcagtgaac ggcactctag taccagggct tcggagcctc 14460
   gtgttgggcg gcaaaagagc tgttaagcga ggagtggtta acctcgtcaa gtatggccgg    14520
   taataaccag agccaaagga agaagaaaaa tgcagctccc atggggaatg gccagtcagt    14580
   caatcagctg tgtcagctgc tgggaacaat gatgaaatcc cagcgccagc ggtctagggg    14640
   gggacaggcc aaaaggaaaa agcctgagaa gccacatttt cccctggctg ctgaagatga    14700
   tgtccggcac cacctcactc aaaccgaacg ttccctttgc ttgcaatcga tccagacggc    14760
   ttttaatcaa ggcgcaggaa ctgcgtcgct ttcacccagt gggaagatcg gttttcaggt    14820
   cgagttcatg ctgccggtca ctcatacggt gcgtttgatt cgcgtgactt ctacatctgc    14880
   tagtcaaggt gtgagttaat ttgatagtca ggtgaatggc cacgattggc gtgtggcctc    14940
   tgagtcacct attcaattag ggcgatcaca tgggggttag acttaattgg cgagaaccat    15000
   gtgaccgaaa tt                                                        15012
<210> 2
   <211> 2369
   <212> PRT
   <213> Porcine reproductive and respiratory syndrome virus
<400> 2
   Met Ser Gly Thr Phe Ser Arg Cys Met Cys Thr Pro Ala Ala Arg Val
   1               5                   10                  15
   Phe Trp Asn Ala Gly Gln Val Phe Cys Thr Arg Cys Leu Ser Ala Arg
               20                  25                  30
   Pro Leu Leu Pro Pro Glu Leu Gln Asp Thr Asp Leu Gly Gly Ile Gly
           35                  40                  45
   Leu Phe Tyr Arg Pro Lys Asp Lys Leu Pro Trp Lys Val Pro Ile Gly
       50                  55                  60
   Ile Pro Gln Val Glu Cys Thr Pro Ser Gly Cys Cys Trp Leu Ser Ala
   65                  70                  75                  80
   Val Phe Pro Leu Ala Arg Met Thr Ser Gly Asn His Asn Phe Val Gln
                   85                  90                  95
   Arg Leu Val Lys Val Ala Asp Val Leu Tyr Arg Asp Gly Cys Leu Thr
               100                 105                 110
   Pro Arg His Leu Arg Glu Leu Gln Val Tyr Glu Arg Gly Cys Asn Trp
           115                 120                 125
   Tyr Pro Ile Thr Gly Pro Val Pro Gly Met Gly Leu Phe Ala Asn Ser
       130                 135                 140
   Met His Val Ser Asp Gln Pro Phe Pro Gly Ala Thr His Val Leu Thr
   145 150 155 160
   Asn Ser Pro Leu Pro Gln Gln Ala Cys Arg Gln Pro Phe Cys Pro Phe
   165 170 175
   Glu Glu Ala His Ser Asp Val Tyr Lys Trp Lys Glu Val Val Val Phe
   180 185 190
   Met Asp Ser Ser Pro Asn Gly Arg Ser Arg Met Met Trp Met Pro Glu
   195 200 205
   Ser Gly Asp Ser Ala Ala Leu Glu Ala Leu Pro Pro Glu Leu Glu Arg
   210 215 220
   Gln Val Glu Ile Leu Ile Arg Ser Phe Pro Ala His His Leu Val Asp
   225 230 235 240
   Leu Thr Asp Trp Glu Leu Thr Glu Ser Pro Glu His Gly Phe Ser Phe
   245 250 255
   Gly Thr Ser Tyr Pro Cys Gly Tyr Leu Ala Gln Asn Pro Tyr Gly Phe
   260 265 270
   Asp Gly Lys Cys Trp Leu Ser Cys Phe Leu Gly Leu Pro Thr Lys Val
   275 280 285
   Trp Arg His Glu Glu Tyr Leu Ala Ser Ala Phe Gly Tyr Gln Thr Lys
   290 295 300
   Trp Gly Val His Gly Lys Tyr Leu Gln Arg Arg Leu Gln Ile Asn Gly
   305 310 315 320
   Val Arg Ala Ala Val Asp Pro Asp Gly Pro Ile His Val Glu Ala Leu
   325 330 335
   Ser Cys Pro Gln Ser Trp Ile Arg His Leu Thr Leu Asp Asp Asp Val
   340 345 350
   Thr Pro Gly Phe Val Arg Leu Met Ser Leu Arg Ile Val Pro Asn Thr
   355 360 365
   Glu Pro Thr Thr Leu Pro Ile Phe Arg Phe Gly Ala His Lys Trp Tyr
   370 375 380
   Gly Ala Ala Gly Lys Arg Ala Arg Ala Lys Arg Ala Ala Lys Gly Lys
   385 390 395 400
   Gly Asp Ser Asn Ser Thr Pro Glu Val Ala Arg Val Ala Pro Thr His
   405 410 415
   Gly Val Ile Thr Tyr Ser Pro Pro Ala Asp Gly Ser Cys Gly Trp His
   420 425 430
   Val Ile Ala Ala Val Met Asn His Met Met Asn Gly Glu Leu Thr Ser
   435 440 445
   Pro Leu Thr Pro Tyr Asn Arg Pro Glu Asp Asp Trp Ala Ser Asp Tyr
   450 455 460
   Asp Leu Val Lys Thr Ile Gln Tyr Leu Gln Leu Pro Ala Thr Val Val
   465 470 475 480
   Arg Ala Arg Ala Cys Pro Asn Ala Lys Tyr Leu Val Lys Leu Asn Gly
   485 490 495
   Val His Trp Glu Val Glu Met Arg Pro Gly Val Ala Pro Arg Ser Leu
   500 505 510
   Pro Arg Glu Cys Val Val Gly Val Cys Ser Glu Gly Cys Val Thr Pro
   515 520 525
   Pro Leu Ser Glu Arg Val Leu Pro Asp Arg Ala Leu Glu Ala Leu Ala
   530 535 540
   Ser Ala Tyr Arg Leu Pro Ser Asp Cys Val Cys Asp Ala Val Ala Asp
   545 550 555 560
   Phe Leu Ser Asn Pro Pro Ser Gln Gly Ser Trp Thr Leu Asp Arg Met
   565 570 575
   Leu Thr Ser Pro Ser Pro Glu Gln Ser Gly Phe Ser Ser Leu Tyr Arg
   580 585 590
   Leu Leu Leu Glu Val Val Pro Gln Lys Cys Gly Ala Thr Glu Gly Ala
   595 600 605
   Phe Val Tyr Ala Val Asp Arg Met Leu Lys Asp Cys Pro Ser Pro Lys
   610 615 620
   Gln Ala Met Ala Leu Leu Gly Lys Ile Lys Ile Pro Ser Ser Lys Ala
   625 630 635 640
   Ser Ser Val Ser Leu Asp Glu Cys Phe Pro Thr Asp Val Leu Thr Val
                   645                 650                 655
   Pro Gly Ser Val Phe Gln Glu Glu Pro Gln Ser Phe Asp Asn Ala Ile
               660                 665                 670
   Ala Leu Cys Ser Leu Glu Lys Gln Lys Glu Thr Glu Gly Ala Ser Pro
           675                 680                 685
   Gly Lys Ala Gln Glu Ser Asp Cys Lys Thr Phe Arg Ser Val Val Pro
       690                 695                 700
   Ala Glu Lys Pro Asn Met Gln Gln Val Pro Leu Val Glu Asp Glu Gln
   705                 710                 715                 720
   Leu Lys Leu Gly Gly Cys Asp Ser Ala Val Ala Thr Val Asp Ser Asp
                   725                 730                 735
   Arg Gly Asn Glu Pro Leu Asp Leu Ser Arg Ser Ala Pro Thr Ala Thr
               740                 745                 750
   Thr Thr Ser Val Gly Gly Arg Ala Pro Asp Asn Pro Gly Pro Asn Thr
           755                 760                 765
   Ser Val Arg Ser Thr Thr Val Gln Glu Ser Val Pro Gly Arg Pro Val
       770                 775                 780
   Pro Arg Leu Val Glu Arg Cys Gly Val Glu Ser Asp Asn Ser Asn Ser
   785                 790                 795                 800
   Pro Leu Asp Leu Ser Lys Ala Gln Thr Pro Asp Arg Pro Leu Asp Leu
                   805                 810                 815
   Ser Leu Ala Ala Trp Pro Val Arg Ala Thr Ala Ser Asp Pro Gly Trp
               820                 825                 830
   Val Asp Gly Arg Arg Glu Pro Val Phe Val Lys Pro Arg Gly Ala Phe
           835                 840                 845
   Ser Asp Ser Glu Ser Val Ile Arg Phe Gly Gly Val Ser Glu Thr Ser
       850                 855                 860
   Ser Ile Ile Glu Phe Asp Arg Thr Glu Glu Thr Pro Val Thr Asp Val
   865                 870                 875                 880
   Pro Ile Asp Leu Thr Thr Ser Asn Glu Thr Leu Ser Gly Ala Asp Pro
                   885                 890                 895
   Phe Glu Leu Ala Glu Pro Lys Arg Pro Arg Leu Ser Ala Gln Ala Leu
               900                 905                 910
   Ile Asp Arg Gly Gly Pro Leu Ala Asp Val His Ala Lys Ile Lys Asn
           915                 920                 925
   Arg Val Tyr Glu Gln Cys Leu Gln Ala Cys Glu Pro Gly Ser Arg Ala
       930                 935                 940
   Thr Pro Ala Thr Lys Glu Trp Leu Asp Lys Met Trp Glu Arg Val Asp
   945                 950                 955                 960
   Met Lys Thr Trp Arg Cys Thr Ser Gln Phe Gln Ala Ala Tyr Ile Leu
                   965                 970                 975
   Gly Pro Leu Lys Phe Leu Pro Asp Met Ile Arg Asp Ala Pro Pro Pro
               980                 985                 990
   Val Pro Arg Lys Asp Arg Ala Cys  Asp Ser Ala Ser Leu  Lys Gln Leu
           995                 1000                1005
   Val Ala  Arg Trp Glu Lys Arg  Leu Asn Ala Val Pro  Leu Gln Gly
       1010                1015                1020
   Pro Val  Glu Pro Glu Leu Gly  Arg Thr Ala Pro Ser  Ser Val Asn
       1025                1030                1035
   Ala Gln  Pro Glu Glu Ala Ile  Pro Ser Asp Gly Pro  Pro Gln Ala
       1040                1045                1050
   Pro Asp  Pro Pro Gly Pro Thr  Ser Ala Gly Arg Gly  Phe Lys Gly
       1055                1060                1065
   Leu Ile  Ser Pro Gly Ile Arg  Leu Ile Gly Ser Ala  Ser Gln Arg
       1070                1075                1080
   Phe Met  Thr Trp Val Phe Glu  Ile Tyr Ser His Leu  Pro Ala Phe
       1085                1090                1095
   Met Leu  Thr Leu Phe Ser Pro  Arg Gly Ser Met Val  Thr Ser Asp
       1100                1105                1110
   Trp Leu  Phe Ala Gly Val Val  Leu Phe Ala Leu Leu  Phe Cys Arg
       1115                1120                1125
   Ser Cys  Pro Ile Leu Gly Cys  Leu Pro Leu Leu Gly  Val Phe Ser
       1130                1135                1140
   Gly Ser Val Arg Cys Val Arg Leu Gly Val Phe Gly Ser Trp Met
   1145 1150 1155
   Ala Phe Ala Val Phe Leu Phe Ser Thr Pro Thr Asn Pro Ile Gly
   1160 1165 1170
   Ser Ser Cys Asp His Asp Ser Pro Glu Cys His Ala Glu Leu Leu
   1175 1180 1185
   Ala Leu Glu Gln Arg Gln Leu Trp Glu Pro Val Arg Ser Leu Val
   1190 1195 1200
   Val Gly Pro Ser Gly Leu Ser Cys Val Ile Leu Gly Lys Leu Leu
   1205 1210 1215
   Gly Gly Ser Arg Tyr Leu Trp His Val Val Leu Arg Leu Cys Met
   1220 1225 1230
   Leu Ala Asp Leu Ala Leu Ser Leu Ile Tyr Val Val Ser Gln Gly
   1235 1240 1245
   Arg Cys Tyr Lys Cys Trp Gly Lys Cys Ile Arg Thr Ala Pro Ala
   1250 1255 1260
   Glu Val Ala Leu Asn Val Phe Pro Phe Ser Arg Ala Thr Arg Ser
   1265 1270 1275
   Ser Leu Val Ser Leu Cys Asp Arg Phe Gln Ala Pro Lys Glu Val
   1280 1285 1290
   Asp Pro Val His Leu Ala Thr Gly Trp Arg Gly Cys Trp Arg Gly
   1295 1300 1305
   Glu Ser Pro Ile His Gln Ala His Gln Lys Pro Ile Ala Tyr Ala
   1310 1315 1320
   Ser Leu Asp Glu Lys Lys Ile Ser Ala Gln Thr Val Val Ala Val
   1325 1330 1335
   Pro Tyr Asp Pro Ser Gln Ala Val Lys Cys Leu Lys Val Leu Gln
   1340 1345 1350
   Ala Gly Gly Ala Ile Val Asp Gln Pro Val Pro Glu Val Ile Arg
   1355 1360 1365
   Val Ser Glu Val Pro Phe Ser Ala Pro Phe Phe Pro Lys Val Pro
   1370 1375 1380
   Val Asn Pro Asp Cys Arg Val Val Val Asp Ser Asp Thr Phe Val
   1385 1390 1395
   Ala Ala Val Arg Cys Gly Tyr Ser Thr Ala Gln Leu Ile Leu Gly
   1400 1405 1410
   Arg Gly Asn Phe Ala Lys Leu Asn Gln Thr Pro Pro Lys Asn Ser
   1415 1420 1425
   Ile Pro Thr Lys Ser Thr Gly Gly Ala Ser Tyr Thr Leu Ala Val
   1430 1435 1440
   Thr Gln Val Ser Val Trp Thr Leu Val His Phe Ile Ile Gly Leu
   1445 1450 1455
   Trp Leu Thr Ser Pro Gln Val Cys Gly Arg Gly Thr Ser Asp Pro
   1460 1465 1470
   Trp Cys Ser Asn Pro Phe Ser Tyr Pro Thr Tyr Gly Pro Gly Val
   1475 1480 1485
   Val Cys Ser Ser Arg Leu Cys Val Ser Ala Asp Gly Val Thr Leu
   1490 1495 1500
   Pro Leu Phe Ser Ala Val Ala Gln Leu Ser Gly Arg Glu Val Gly
   1505 1510 1515
   Val Phe Ile Leu Val Leu Val Ser Leu Ile Ser Leu Ala His Arg
   1520 1525 1530
   Leu Ala Leu Lys Ala Asp Met Leu Val Val Phe Ser Ala Phe Cys
   1535 1540 1545
   Ala Tyr Ala Trp Pro Met Ser Ser Trp Leu Ile Cys Phe Phe Pro
   1550 1555 1560
   Ile Leu Leu Arg Trp Val Thr Leu His Pro Leu Thr Met Leu Trp
   1565 1570 1575
   Val His Ser Phe Leu Val Phe Cys Leu Pro Ala Ala Gly Val Leu
   1580 1585 1590
   Ser Leu Gly Ile Ser Gly Phe Leu Trp Ala Val Gly Arg Phe Thr
   1595 1600 1605
   Gln Val  Ala Gly Ile Ile Thr  Pro Tyr Asp Ile His  Gln Tyr Thr
       1610                 1615                 1620
   Ser Gly  Pro Arg Gly Ala Ala  Ala Val Ala Thr Ala  Pro Glu Gly
       1625                 1630                 1635
   Thr Tyr  Met Ala Ala Val Arg  Arg Ala Ala Leu Thr  Gly Arg Thr
       1640                 1645                 1650
   Leu Ile  Phe Thr Pro Ser Ala  Val Gly Ser Leu Leu  Glu Gly Ala
       1655                 1660                 1665
   Phe Arg  Thr Asn Lys Pro Cys  Leu Asn Thr Val Asn  Val Val Gly
       1670                 1675                 1680
   Ser Ser  Leu Gly Ser Gly Gly  Val Phe Thr Val Gly  Gly Ser Lys
       1685                 1690                 1695
   Ile Ile  Ile Thr Ala Thr His  Val Leu Asn Gly Asp  Ala Ala Arg
       1700                 1705                 1710
   Val Thr  Gly Asp Ser Tyr Asn  Arg Met His Thr Phe  Lys Thr Asn
       1715                 1720                 1725
   Gly Asp  Tyr Ala Trp Ser Arg  Ala Asp Asp Trp Gln  Gly Val Ala
       1730                 1735                 1740
   Pro Ala  Val Lys Val Ala Lys  Arg Tyr Arg Gly Arg  Ala Tyr Trp
       1745                 1750                 1755
   Gln Thr  Ser Thr Gly Val Glu  Pro Gly Ile Ile Gly  Glu Gly Phe
       1760                 1765                 1770
   Ala Phe  Cys Phe Thr Asn Cys  Gly Asp Ser Gly Ser  Pro Val Ile
       1775                 1780                 1785
   Ser Glu  Thr Gly Asp Leu Ile  Gly Val His Thr Gly  Ser Asn Lys
       1790                 1795                 1800
   Leu Gly  Ser Gly Leu Val Thr  Thr Pro Glu Gly Glu  Thr Cys Ser
       1805                 1810                 1815
   Ile Lys  Glu Thr Lys Leu Ser  Glu Leu Ser Arg Tyr  Phe Ala Gly
       1820                 1825                 1830
   Pro Ser  Val Pro Leu Gly Asp  Val Arg Leu Ser Pro  Ala Ile Ile
       1835                 1840                 1845
   Pro Asp Val Ala Phe Ile Pro Ser Asp Leu Ala Ser Leu Leu Ala
   1850 1855 1860
   Ser Val Pro Val Met Glu Gly Gly Leu Ser Thr Val Gln Leu Leu
   1865 1870 1875
   Cys Val Phe Phe Leu Leu Trp Arg Met Met Gly His Ala Trp Thr
   1880 1885 1890
   Pro Ile Ile Ala Val Ser Phe Phe Leu Leu Asn Glu Ile Leu Pro
   1895 1900 1905
   Ala Val Leu Val Arg Ala Val Phe Ser Phe Ala Leu Phe Val Leu
   1910 1915 1920
   Ala Trp Ala Thr Pro Trp Ser Ala Gln Val Leu Met Ile Arg Leu
   1925 1930 1935
   Leu Thr Ala Ala Leu Asn Arg Asn Arg Leu Ser Leu Ala Phe Tyr
   1940 1945 1950
   Ala Leu Gly Gly Val Val Gly Leu Ala Ala Glu Ile Gly Thr Phe
   1955 1960 1965
   Ala Gly Lys Leu Thr Glu Leu Ser Gln Val Leu Ser Thr Tyr Cys
   1970 1975 1980
   Phe Leu Pro Arg Val Ile Ala Met Thr Ser Cys Val Pro Val Ile
   1985 1990 1995
   Ile Ile Gly Gly Leu His Ala Leu Gly Val Ile Leu Trp Leu Phe
   2000 2005 2010
   Lys His Arg Cys Leu His Asn Met Leu Val Gly Asp Gly Ser Phe
   2015 2020 2025
   Ser Ser Ala Phe Phe Leu Arg Tyr Phe Ala Glu Gly Asn Leu Arg
   2030 2035 2040
   Lys Gly Val Ser Gln Ser Cys Gly Met Ser Asn Glu Ser Leu Thr
   2045 2050 2055
   Ala Ala Leu Ala Cys Lys Leu Ser Gln Ala Asp Leu Asp Phe Leu
   2060 2065 2070
   Ser Gly Leu Thr Asn Phe Lys Cys Phe Val Ser Ala Ser Asn Met
   2075 2080 2085
   Lys Asn Ala Ala Gly Gln Tyr Ile Glu Ala Ala Tyr Ala Lys Ala
   2090 2095 2100
   Leu Arg Gln Glu Leu Ala Ser Leu Val Gln Val Asp Lys Met Lys
   2105 2110 2115
   Gly Val Leu Ser Lys Leu Glu Ala Phe Ala Glu Met Ala Thr Pro
   2120 2125 2130
   Ser Leu Asp Thr Gly Asp Val Val Val Leu Leu Gly Gln His Pro
   2135 2140 2145
   His Gly Ser Ile Leu Asp Ile Asn Val Gly Thr Glu Arg Lys Thr
   2150 2155 2160
   Val Ser Val Gln Glu Thr Arg Ser Leu Gly Gly Ser Lys Phe Ser
   2165 2170 2175
   Val Cys Thr Val Val Ser Asn Thr Pro Val Asp Ala Leu Thr Gly
   2180 2185 2190
   Ile Pro Leu Gln Thr Pro Thr Pro Leu Phe Glu Asn Gly Pro Arg
   2195 2200 2205
   His Arg Ser Glu Asp Asp Asp Leu Lys Val Glu Arg Met Lys Lys
   2210 2215 2220
   His Cys Val Ser Leu Gly Phe His Asn Ile Asn Gly Lys Ile Tyr
   2225 2230 2235
   Cys Lys Val Trp Asp Lys Ser Thr Gly Asp Thr Phe Tyr Thr Asp
   2240 2245 2250
   Asp Ser Arg Tyr Thr Gln Asp His Ala Phe Gln Asp Arg Ser Ala
   2255 2260 2265
   Asp Tyr Arg Asp Arg Asp Tyr Glu Gly Val Gln Ile Ala Pro Gln
   2270 2275 2280
   Gln Gly Phe Asp Pro Met Ser Glu Thr Pro Val Gly Thr Val Val
   2285 2290 2295
   Ile Gly Gly Ile Thr Tyr Ser Arg Tyr Leu Val Lys Gly Lys Glu
   2300 2305 2310
   Val Leu Ile Pro Lys Pro Asp Asn His Phe Glu Ala Ala Lys Leu
           2315                2320                2325
   Ser Leu  Glu Gln Ala Leu Ala  Gly Met Gly Gln Thr  Cys Asp Leu
       2330                2335                2340
   Thr Ala  Ala Glu Val Glu Lys  Leu Lys Arg Ile Ile  Ser Gln Leu
       2345                2350                2355
   Gln Gly  Leu Thr Thr Glu Gln  Ala Leu Asn Cys
       2360                2365
<210> 3
   <211> 1460
   <212> **PRT**
   <213> Porcine reproductive and respiratory syndrome virus
<400> 3
   Thr Gly Phe Lys Leu Leu Ala Ala Ser Gly Leu Thr Arg Cys Gly Arg
   1               5                   10                  15
   Gly Gly Leu Val Val Thr Glu Thr Ala Val Lys Ile Val Lys Tyr His
               20                  25                  30
   Ser Arg Thr Phe Thr Leu Gly Pro Leu Asp Leu Lys Val Thr Ser Glu
           35                  40                  45
   Ala Glu Val Lys Lys Ser Thr Glu Gln Gly His Ala Val Val Ala Asn
       50                  55                  60
   Leu Cys Ser Gly Val Val Leu Met Arg Pro His Pro Pro Ser Leu Val
   65                  70                  75                  80
   Asp Val Leu Leu Ile Pro Gly Leu Asp Thr Ala Pro Gly Ile Gln Pro
                   85                  90                  95
   Gly His Gly Ala Gly Asn Met Gly Val Asn Gly Ala Ile Trp Asp Phe
               100                 105                 110
   Glu Thr Ala Pro Thr Lys Ala Glu Leu Glu Leu Ser Lys Gln Ile Ile
           115                 120                 125
   Gln Ala Cys Glu Val Arg Arg Gly Asp Ala Pro Asn Leu Gln Leu Pro
       130                 135                 140
   Tyr Lys Leu His Pro Val Arg Gly Asp Pro Glu Arg Arg Asn Gly Arg
   145                 150                 155                 160
   Leu Ile Asn Thr Arg Phe Gly Asp Leu Pro Tyr Lys Thr Pro Gln Asp
   165 170 175
   Thr Arg Ser Ala Ile His Ala Ala Cys Cys Leu His Pro Asn Gly Val
   180 185 190
   Pro Val Ser Asp Gly Lys Ser Thr Leu Gly Thr Thr Leu Gln Arg Gly
   195 200 205
   Phe Glu Leu Tyr Val Pro Thr Val Pro Tyr Ser Val Leu Glu Tyr Leu
   210 215 220
   Asp Ser Arg Pro Asp Thr Pro Phe Met Cys Thr Lys His Gly Thr Ser
   225 230 235 240
   Glu Ala Ala Ala Glu Asp Leu Gln Lys Tyr Asn Leu Ser Thr Gln Gly
   245 250 255
   Phe Val Leu Pro Gly Val Leu Arg Leu Val Arg Arg Phe Ile Phe Gly
   260 265 270
   His Ile Gly Lys Ala Pro Pro Leu Tyr Leu Pro Ser Thr Tyr Pro Ala
   275 280 285
   Lys Asn Ser Met Ala Gly Ile Asn Gly Gln Arg Phe Pro Thr Lys Asp
   290 295 300
   Val Gln Ser Ile Pro Glu Val Asp Glu Met Cys Ala Arg Ala Val Lys
   305 310 315 320
   Asp Asn Trp Gln Thr Thr Thr Pro Cys Thr Leu Lys Lys Gln Tyr Cys
   325 330 335
   Ser Lys Pro Lys Thr Arg Thr Ile Leu Gly Thr Asn Asn Phe Ile Ala
   340 345 350
   Leu Ala His Arg Ser Ala Leu Ser Gly Val Thr Gln Ala Phe Met Lys
   355 360 365
   Lys Ala Trp Lys Ser Pro Ile Ala Leu Gly Lys Asn Lys Phe Lys Glu
   370 375 380
   Leu His Cys Ala Val Ala Gly Arg Cys Leu Glu Ala Asp Leu Ala Ser
   385 390 395 400
   Cys Asp Arg Ser Thr Pro Ala Ile Val Arg Trp Phe Thr Ala Asn Leu
   405 410 415
   Leu Tyr Glu Leu Ala Gly Cys Glu Glu Tyr Leu Pro Ser Tyr Val Leu
               420                 425                 430
   Asn Cys Cys His Asp Leu Val Ala Thr Gln Asp Gly Ala Phe Thr Lys
           435                 440                 445
   Arg Gly Gly Leu Ser Ser Gly Asp Pro Val Thr Ser Val Ser Asn Thr
       450                 455                 460
   Val Tyr Ser Leu Val Ile Tyr Ala Gln His Met Val Leu Ser Ala Leu
   465                 470                 475                 480
   Lys Met Gly His Glu Ile Gly Leu Lys Phe Leu Glu Glu Gln Leu Arg
                   485                 490                 495
   Phe Glu Asp Leu Ile Glu Ile Gln Pro Met Leu Val Tyr Ser Asp Asp
               500                 505                 510
   Leu Val Leu Tyr Ala Glu Lys Pro Thr Phe Pro Asn Tyr His Trp Trp
           515                 520                 525
   Val Glu His Leu Asp Leu Met Leu Gly Phe Lys Thr Asp Pro Lys Lys
       530                 535                 540
   Thr Ile Ile Thr Asp Lys Pro Ser Phe Leu Gly Cys Arg Ile Glu Ala
   545                 550                 555                 560
   Gly Arg Gln Leu Val Pro Asn Arg Asp Arg Ile Leu Ala Ala Leu Ala
                   565                 570                 575
   Tyr His Met Lys Ala Gln Asn Ala Ser Glu Tyr Tyr Ala Ser Ala Ala
               580                 585                 590
   Ala Ile Leu Met Asp Ser Cys Ala Cys Ile Asp Tyr Asp Pro Glu Trp
           595                 600                 605
   Tyr Glu Asp Leu Ile Cys Gly Ile Ala Arg Cys Ala Arg Gln Asp Gly
       610                 615                 620
   Tyr Ser Phe Pro Gly Pro Ala Phe Phe Met Ser Met Trp Glu Lys Leu
   625                 630                 635                 640
   Lys Ser His Asn Glu Gly Lys Lys Phe Arg His Cys Gly Ile Cys Asp
                   645                 650                 655
   Ala Lys Ala Asp His Ala Ser Ala Cys Gly Leu Asp Leu Cys Leu Phe
               660                 665                 670
   His Ser His Phe His Gln His Cys Pro Val Thr Leu Ser Cys Gly His
           675                 680                 685
   His Ala Gly Ser Lys Glu Cys Pro Gln Cys Gln Ser Pro Val Gly Ala
       690                 695                 700
   Gly Lys Ser Pro Leu Asp Thr Val Leu Gln Gln Ile Pro Tyr Lys Pro
   705                 710                 715                 720
   Pro Arg Thr Val Ile Met Lys Val Asn Asn Lys Thr Thr Ala Leu Asp
                   725                 730                 735
   Pro Gly Arg Tyr Gln Ser Arg Arg Gly Leu Val Ala Val Lys Arg Gly
               740                 745                 750
   Ile Ala Gly Asn Glu Val Asp Leu Ala Asp Gly Asp Tyr Gln Val Val
           755                 760                 765
   Pro Leu Leu Pro Thr Cys Lys Asp Ile Asn Met Val Lys Val Ala Cys
       770                 775                 780
   Asn Val Leu Leu Ser Lys Phe Ile Val Gly Pro Pro Gly Ser Gly Lys
   785                 790                 795                 800
   Thr Thr Trp Leu Leu Asn Gln Val Gln Glu Asp Val Val Ile Tyr Thr
                   805                 810                 815
   Pro Thr His Gln Thr Met Phe Asp Ile Val Ser Ala Leu Lys Val Cys
               820                 825                 830
   Arg Tyr Ser Ile Pro Gly Ala Ser Gly Leu Pro Phe Pro Pro Pro Ala
           835                 840                 845
   Arg Ser Gly Pro Trp Val Arg Leu Val Ala Ser Gly His Ile Pro Gly
       850                 855                 860
   Arg Val Ser Tyr Leu Asp Glu Ala Gly Tyr Cys Asn His Leu Asp Ile
   865                 870                 875                 880
   Leu Arg Leu Leu Ser Lys Thr Pro Leu Val Cys Leu Gly Asp Leu Gln
                   885                 890                 895
   Gln Leu His Pro Val Gly Phe Asp Ser Cys Cys Tyr Val Phe Asn Gln
               900                 905                 910
   Met Pro His Lys Gln Leu Thr Thr Ile Tyr Arg Phe Gly Pro Asn Ile
           915                 920                 925
   Cys Ala Ala Ile Gln Pro Cys Tyr Lys Glu Lys Leu Glu Ser Lys Ala
       930                 935                 940
   Arg Asn Thr Arg Val Val Phe Thr Ile Arg Pro Val Ala Tyr Gly Gln
   945                 950                 955                 960
   Val Leu Thr Pro Phe His Lys Asp Arg Val Gly Ser Ala Ile Thr Ile
                   965                 970                 975
   Asp Ser Ser Gln Gly Ala Thr Phe Asp Val Val Thr Leu His Leu Pro
               980                 985                 990
   Thr Pro Arg Ser Leu Asn Lys Ser  Arg Ala Leu Val Ala  Ile Thr Arg
           995                 1000                 1005
   Ala Arg  His Gly Leu Phe Ile  Tyr Asp Pro His Asn  Gln Leu Gln
       1010                 1015                 1020
   Glu Phe  Phe Asn Leu Thr Pro  Glu Arg Thr Asp Cys  Asn Leu Val
       1025                 1030                 1035
   Phe Asn  Arg Gly Gly Glu Leu  Val Val Leu Asn Ser  Asp Asn Ala
       1040                 1045                 1050
   Val Thr  Thr Val Ala Lys Val  Leu Glu Ala Gly Pro  Ser Gln Phe
       1055                 1060                 1065
   Arg Val  Ser Asp Pro Arg Cys  Lys Ser Leu Leu Ala  Ala Cys Ser
       1070                 1075                 1080
   Val Ser  Leu Glu Gly Ser Cys  Met Pro Leu Pro Gln  Val Ala His
       1085                 1090                 1095
   Asn Leu  Gly Phe Tyr Phe Ser  Pro Asp Ser Pro Ala  Phe Ala Pro
       1100                 1105                 1110
   Leu Pro  Lys Glu Leu Ala Pro  His Trp Pro Val Val  Thr Cys Gln
       1115                 1120                 1125
   Asn Asn  Gln Ala Trp Pro Asp  Arg Leu Val Ala Ser  Met Arg Pro
       1130                 1135                 1140
   Ile Asp  Ala Arg Tyr Ser Lys  Pro Met Val Gly Ala  Gly Tyr Val
       1145                 1150                 1155
   Val Gly  Pro Ser Thr Phe Leu  Gly Thr Pro Gly Val  Val Ser Tyr
   1160 1165 1170
   Tyr Leu Thr Leu Tyr Ile Lys Gly Glu Pro Arg Ala Leu Pro Glu
   1175 1180 1185
   Thr Leu Val Ser Thr Gly Arg Ile Ala Thr Asp Cys Arg Glu Tyr
   1190 1195 1200
   Leu Asp Ala Ala Glu Glu Glu Ala Ala Lys Glu Leu Pro His Ala
   1205 1210 1215
   Phe Ile Gly Asp Val Lys Gly Thr Thr Val Gly Gly Cys His His
   1220 1225 1230
   Ile Thr Ser Lys Tyr Leu Pro Arg Ser Leu Pro Lys Asp Ser Val
   1235 1240 1245
   Ala Val Val Gly Val Ser Ser Pro Gly Lys Ala Ala Lys Ala Val
   1250 1255 1260
   Cys Thr Leu Thr Asp Val Tyr Leu Pro Asp Leu Arg Pro Tyr Leu
   1265 1270 1275
   Gln Pro Glu Thr Ala Ser Lys Cys Trp Lys Leu Lys Leu Asp Phe
   1280 1285 1290
   Arg Asp Val Arg Leu Met Val Trp Lys Gly Ala Thr Ala Tyr Phe
   1295 1300 1305
   Gln Leu Glu Gly Leu Thr Trp Ser Ala Leu Pro Asp Tyr Ala Arg
   1310 1315 1320
   Phe Ile Gln Leu Pro Lys Asp Ala Val Val Tyr Ile Asp Pro Cys
   1325 1330 1335
   Ile Gly Pro Ala Thr Ala Asn Arg Lys Val Val Arg Thr Thr Asp
   1340 1345 1350
   Trp Arg Ala Asp Leu Ala Val Thr Pro Tyr Asp Tyr Gly Ala Gln
   1355 1360 1365
   Thr Ile Leu Thr Thr Ala Trp Phe Glu Asp Leu Gly Pro Gln Trp
   1370 1375 1380
   Lys Ile Leu Gly Leu Gln Pro Phe Lys Arg Ala Phe Gly Phe Glu
   1385 1390 1395
   Asn Thr Glu Asp Trp Ala Ile Leu Ala Arg Arg Met Asn Asp Gly
   1400 1405 1410
   Arg Asp Tyr Thr Asp Tyr Asn Trp Thr Cys Val Arg Glu Arg Pro
   1415 1420 1425
   His Ala Ile Tyr Gly Arg Ala Arg Asp His Thr Tyr His Phe Ala
   1430 1435 1440
   Pro Gly Ala Glu Leu Gln Val Glu Leu Gly Lys Pro Arg Leu Pro
   1445 1450 1455
   Pro Glu
   1460
<210> 4
   <211> 249
   <212> PRT
   <213> Porcine reproductive and respiratory syndrome virus
<400> 4
   Met Gln Trp Gly His Cys Gly Val Lys Ser Pro Ser Cys Leu Trp Met
   1 5 10 15
   Pro Leu Leu Ser Ser Leu Ser Val Trp Leu Thr Leu Ser Ser Phe Leu
   20 25 30
   Pro Tyr Cys Ser Gly Ser Pro Ser Gln Asp Gly Tyr Trp Ser Phe Tyr
   35 40 45
   Ser Glu Trp Phe Ala Pro Arg Phe Ser Val Arg Ala Leu Pro Phe Thr
   50 55 60
   Leu Pro Asn Tyr Arg Arg Ser Tyr Glu Gly Leu Leu Pro Asn Cys Arg
   65 70 75 80
   Pro Asp Val Pro Gln Phe Ala Ile Lys His Pro Leu Gly Ile Leu Trp
   85 90 95
   His Met Arg Val Ser His Leu Ile Asp Glu Met Val Ser Arg Arg Val
   100 105 110
   Tyr Gln Thr Met Glu His Ser Gly Gln Ala Ala Trp Lys Gln Val Val
   115 120 125
   Ala Glu Ala Thr Leu Thr Lys Leu Ser Gln Leu Asp Ile Val Thr His
   130 135 140
   Phe Gln His Leu Ala Ala Val Glu Ala Asp Ser Cys Arg Phe Leu Ser
   145                 150                 155                 160
   Ser Arg Leu Val Met Leu Lys Asn Leu Ala Val Gly Asn Val Ser Leu
                   165                 170                 175
   Leu Tyr Asn Thr Thr Leu Asp Arg Val Glu Leu Ile Phe Pro Thr Pro
               180                 185                 190
   Gly Thr Arg Pro Lys Leu Thr Asp Phe Arg Gln Trp Leu Ile Ser Val
           195                 200                 205
   His Ala Ser Ile Phe Ser Ser Val Ala Ser Ser Val Thr Leu Phe Val
       210                 215                 220
   Val Leu Trp Leu Arg Val Pro Ile Leu Arg Tyr Val Phe Gly Phe His
   225                 230                 235                 240
   Trp Pro Thr Ala Thr Arg Arg Leu Ser
                   245
<210> 5
   <211> 70
   <212> PRT
   <213> Porcine reproductive and respiratory syndrome virus
<400> 5
   Met Gly Ser Leu Trp Ser Lys Ile Thr Gln Leu Phe Val Asp Ala Phe
   1               5                   10                  15
   Thr Glu Phe Leu Ile Ser Val Val Asp Ile Ile Ile Phe Leu Ala Ile
               20                  25                  30
   Leu Phe Gly Phe Thr Val Ala Gly Trp Leu Leu Val Phe Leu Leu Arg
           35                  40                  45
   Val Val Cys Ser Ala Ile Leu Arg Ser Arg Thr Ala Val His Ser Pro
       50                  55                  60
   Glu Leu Ser Lys Val Leu
   65                  70
<210> 6
   <211> 255
   <212> PRT
   <213> Porcine reproductive and respiratory syndrome virus
<400> 6
   Met Ala His Gln Cys Ala Arg Leu His Leu Leu Phe Cys Gly Leu Val
   1               5                   10                  15
   Ser His Phe Val Arg Gly Ala Leu Ala Ser Gly Pro Asn Ser Thr Leu
               20                  25                  30
   Cys Phe Trp Phe Pro Leu Ala His Gly Asn Thr Ser Phe Glu Leu Thr
           35                  40                  45
   Ile Asn Tyr Thr Val Cys Lys Pro Cys Leu Thr Arg Asp Ala Ala Arg
       50                  55                  60
   Gln Arg Leu Glu Pro Gly Arg Asn Met Trp Cys Lys Ile Gly His Asp
   65                  70                  75                  80
   Arg Cys Glu Asp Arg Asp His Asp Glu Leu Ser Met Thr Ile Pro Pro
                   85                  90                  95
   Gly Tyr Asp Asn Leu Lys Leu Glu Gly Tyr Tyr Ala Trp Leu Ala Phe
               100                 105                 110
   Leu Ser Phe Ser Tyr Ala Ala Gln Phe His Pro Glu Leu Phe Gly Ile
           115                 120                 125
   Gly Asn Val Ser Arg Val Phe Val Asp Lys Val His Gln Phe Val Cys
       130                 135                 140
   Ala Val His Asp Gly Gln Asn Ser Thr Val Ser Asn Gly His Asn Ile
   145                 150                 155                 160
   Ser Ala Leu Tyr Ala Ala Tyr Tyr His His Gln Ile Asp Gly Gly Asn
                   165                 170                 175
   Trp Phe His Leu Glu Trp Leu Arg Pro Phe Phe Ser Ser Trp Leu Val
               180                 185                 190
   Leu Asn Ile Ser Trp Phe Leu Arg Arg Ser Pro Ala Ser Pro Val Ser
           195                 200                 205
   Arg His Ile Tyr Gln Ile Leu Arg Pro Thr Arg Gln Arg Leu Pro Val
       210                 215                 220
   Ser Trp Ser Phe Lys Thr Ser Ala Ala Ser Ser Leu Met Gly Val Gln
   225                 230                 235                 240
   Lys Arg Lys Ala Pro Ala Phe Met Gly Ser His Leu Asn Val Val
                   245                 250                 255
<210> 7
   <211> 183
   <212> PRT
   <213> Porcine reproductive and respiratory syndrome virus
<400> 7
   Met Val Ala Ala Ile Leu Phe Leu Leu Ala Gly Ala Gln His Phe Met
   1               5                   10                  15
   Val Ser Glu Ala Phe Ala Cys Lys Pro Cys Phe Ser Thr His Leu Ser
               20                  25                  30
   Asp Ile Lys Thr Asn Thr Thr Ala Ala Ala Gly Phe Met Val Leu Gln
           35                  40                  45
   Asp Ile Ser Cys Leu Gln Pro His Gly Gly Ser Glu Thr Gln Gly Thr
       50                  55                  60
   Ser Ile His Gly Lys Pro Ser Gln Cys Arg Val Ala Val Gly Ile Pro
   65                  70                  75                  80
   Gln Tyr Ile Thr Val Thr Ala Asn Val Thr Asp Glu Ser Tyr Leu Tyr
                   85                  90                  95
   Asn Ala Asp Leu Leu Met Leu Ser Ala Cys Leu Phe Tyr Ala Ser Glu
               100                 105                 110
   Met Ser Glu Lys Gly Phe Lys Val Ile Phe Gly Asn Val Ser Gly Val
           115                 120                 125
   Val Ser Ala Cys Val Asn Phe Thr Asp Tyr Val Ala His Val Thr Gln
       130                 135                 140
   His Thr Gln Gln His His Leu Val Ile Asp His Val Arg Leu Leu His
   145                 150                 155                 160
   Phe Leu Thr Pro Ser Thr Met Arg Trp Ala Thr Thr Ile Ala Cys Leu
                   165                 170                 175
   Phe Ala Ile Leu Leu Ala Ile
               180
<210> 8
   <211> 201
   <212> PRT
   <213> Porcine reproductive and respiratory syndrome virus
<400> 8
   Met Arg Cys Ser His Lys Leu Gly Cys Ile Leu Thr Pro Tyr Pro Cys
   1               5                   10                  15
   Cys Cys Trp Leu Phe Leu Leu Cys Thr Gly Leu Ser Trp Ser Phe Ala
               20                  25                  30
   Asp Gly Asn Gly Asn Ser Ser Thr Tyr Gln Tyr Ile Tyr Asn Leu Thr
           35                  40                  45
   Ile Cys Glu Leu Asn Gly Thr Ala Trp Leu Ser Asn His Phe Gln Trp
       50                  55                  60
   Ala Val Glu Thr Phe Val Leu Tyr Pro Val Val Thr His Ile Leu Ser
   65                  70                  75                      80
   Leu Gly Phe Leu Thr Thr Ser His Phe Phe Asp Thr Leu Gly Leu Gly
                   85                  90                  95
   Ala Val Ser Val Thr Gly Phe Cys His Thr Arg Tyr Val Leu Ser Ser
               100                 105                 110
   Val Tyr Gly Val Cys Ala Leu Ala Ala Phe Ile Cys Phe Thr Ile Arg
           115                 120                 125
   Ala Ala Lys Asn Cys Met Ala Cys Arg Tyr Ala Arg Thr Arg Phe Thr
       130                 135                 140
   Asn Phe Ile Val Asp Asp Arg Gly Arg Ile His Arg Trp Lys Ser Pro
   145                 150                 155                     160
   Ile Val Val Glu Lys Leu Gly Lys Ala Glu Ile Gly Asn Asp Leu Ile
                   165                 170                 175
   Thr Ile Lys His Val Val Leu Glu Gly Val Lys Ala Gln Pro Leu Thr
               180                 185                 190
   Arg Thr Ser Ala Glu Gln Trp Glu Ala
           195                 200
<210> 9
   <211> 173
   <212> PRT
   <213> Porcine reproductive and respiratory syndrome virus
<400> 9
   Met Gly Gly Ile Asp Asp Phe Cys Tyr Asp Pro Thr Ala Thr Gln Lys
   1               5                   10                  15
   Leu Val Leu Ala Phe Ser Ile Thr Tyr Thr Pro Ile Met Ile Tyr Ala
               20                  25                  30
   Leu Lys Val Ser Arg Gly Arg Leu Leu Gly Leu Leu His Ile Leu Ile
           35                  40                  45
   Phe Leu Asn Cys Ser Phe Thr Phe Gly Tyr Met Thr Tyr Val His Phe
       50                  55                  60
   Gln Ser Thr Asn Arg Val Ala Leu Thr Met Gly Ala Val Val Ala Leu
   65                  70                  75                  80
   Leu Trp Gly Ile Tyr Ser Phe Ile Glu Ser Trp Lys Phe Ile Thr Ser
                   85                  90                  95
   Arg Cys Arg Leu Cys Cys Leu Gly Arg Arg Tyr Ile Leu Ala Pro Ala
               100                 105                 110
   His His Val Glu Ser Ala Ala Gly Leu His Ser Ile Pro Ala Ser Gly
           115                 120                 125
   Asn Arg Ala Tyr Ala Val Arg Lys Pro Gly Leu Thr Ser Val Asn Gly
       130                 135                 140
   Thr Leu Val Pro Gly Leu Arg Ser Leu Val Leu Gly Gly Lys Arg Ala
   145                 150                 155                 160
   Val Lys Arg Gly Val Val Asn Leu Val Lys Tyr Gly Arg
                   165                 170
<210> 10
   <211> 128
   <212> PRT
   <213> Porcine reproductive and respiratory syndrome virus
<400> 10
   Met Ala Gly Asn Asn Gln Ser Gln Arg Lys Lys Lys Asn Ala Ala Pro
   1               5                   10                  15
   Met Gly Asn Gly Gln Ser Val Asn Gln Leu Cys Gln Leu Leu Gly Thr
               20                  25                  30
   Met Met Lys Ser Gln Arg Gln Arg Ser Arg Gly Gly Gln Ala Lys Arg
           35                  40                  45
   Lys Lys Pro Glu Lys Pro His Phe Pro Leu Ala Ala Glu Asp Asp Val
       50                  55                  60
   Arg His His Leu Thr Gln Thr Glu Arg Ser Leu Cys Leu Gln Ser Ile
   65                  70                  75                  80
   Gln Thr Ala Phe Asn Gln Gly Ala Gly Thr Ala Ser Leu Ser Pro Ser
                   85                  90                  95
   Gly Lys Ile Gly Phe Gln Val Glu Phe Met Leu Pro Val Thr His Thr
               100                 105                 110
   Val Arg Leu Ile Arg Val Thr Ser Thr Ser Ala Ser Gln Gly Val Ser
           115                 120                 125
<210> 11
   <211> 56
   <212> DNA
   <213> Artificial Sequence
<220>
   <223> synthetic primer
<400> 11
   caggaaacag ctatgacacc tgatctctag aaacgttttt tttttttttt tttttt 56
<210> 12
   <211> 21
   <212> DNA
   <213> Artificial Sequence
<220>
   <223> synthetic primer
<400> 12
   tgatgtgtag ggtattcccc c 21
<210> 13
   <211> 20
   <212> DNA
   <213> Artificial Sequence
<220>
   <223> synthetic primer
<400> 13
   cgtgacccac cgagtaactt 20
<210> 14
   <211> 20
   <212> DNA
   <213> Artificial Sequence
<220>
   <223> synthetic primer
<400> 14
   tcttgagcag cgccaacttt 20
<210> 15
   <211> 19
   <212> DNA
   <213> Artificial Sequence
<220>
   <223> synthetic primer
<400> 15
   tcataccact cagggtcat 19
<210> 16
   <211> 21
   <212> DNA
   <213> Artificial Sequence
<220>
   <223> synthetic primer
<400> 16
   ccaggcgtty atgaagaaag c 21
<210> 17
   <211> 23
   <212> DNA
   <213> Artificial Sequence
<220>
   <223> synthetic primer
<400> 17
   aacaagcctt cataggacct tcg 23
<210> 18
   <211> 22
   <212> DNA
   <213> Artificial Sequence
<220>
   <223> synthetic primer
<400> 18
   tactgttcgg gttcaccgtc gc 22
<210> 19
   <211> 19
   <212> DNA
   <213> Artificial Sequence
<220>
   <223> synthetic primer
<400> 19
   aatttcggtc acatggttc 19
<210> 20
   <211> 15111
   <212> DNA
   <213> Lelystad virus
<400> 20
   atgatgtgta gggtattccc cctacataca cgacacttct agtgtttgtg taccttggag 60
   gcgtgggtac agccccgccc caccccttgg cccctgttct agcccaacag gtatccttct 120
   ctctcggggc gagtgcgccg cctgctgctc ccttgcagcg ggaaggacct cccgagtatt 180
   tccggagagc acctgcttta cgggatctcc accctttaac catgtctggg acgttctccc 240
   ggtgcatgtg caccccggct gcccgggtat tttggaacgc cggccaagtc ttttgcacac 300
   ggtgtctcag tgcgcggtct cttctctctc cagagcttca ggacactgac ctcggtgcag 360
   ttggcttgtt ttacaagcct agggacaagc ttcactggaa agtccctatc ggcatccctc 420
   aggtggaatg tactccatcc gggtgctgtt ggctctcagc tgttttccct ttggcgcgta 480
   tgacctccgg caatcacaac ttcctccaac gacttgtgaa ggttgctgat gttttgtacc 540
   gtgacggttg cttggcacct cgacaccttc gtgaactcca agtttacgag cgcggctgca 600
   actggtaccc gatcacgggg cccgtgcccg ggatgggttt gtttgcgaac tccatgcacg 660
   tatccgacca gccgttccct ggtgccaccc atgtgttgac taactcgcct ttgcctcaac 720
   aggcttgtcg gcagccgttc tgtccatttg aggaggctca ttctagcgtg tacaggtgga 780
   agaaatttgt ggttttcacg gactcctccc tcaacggtcg atctcgcatg atgtggacgc 840
   cggaatccga tgattcagcc gccctggagg tactaccgcc tgagttagaa cgtcaggtcg 900
   aaatcctcat tcggagtttt cctgctcatc accctgtcga cctggccgac tgggagctca 960
   ctgagtcccc tgagaacggt ttttccttca acacgtctca ttcttgcggt caccttgtcc 1020
   agaaccccga cgtgtttgat ggcaagtgct ggctctcctg ctttttgggc cagtcggtcg 1080
   aagtgcgctg ccatgaggaa catctagctg acgccttcgg ttaccaaacc aagtggggcg 1140
   tgcatggtaa gtacctccag cgcaggcttc aagttcgcgg cattcgtgct gtagtcgatc 1200
   ctgatggtcc cattcacgtt gaagcgctgt cttgccccca gtcttggatc aggcacctga 1260
   ctctggatga tgatgtcacc ccaggattcg ttcgcctgac atcccttcgc attgtgccga 1320
   acacagagcc taccacttcc cggatctttc ggtttggagc gcataagtgg tatggcgctg 1380
   ccggcaaacg ggctcgtgct aagcgtgccg ctaaaagtga gaaggattcg gctcccaccc 1440
   ccaaggttgc cctgccggtc cccacctgtg gaattaccac ctactctcca ccgacagacg 1500
   ggtcttgtgg ttggcatgtc cttgccgcca taatgaaccg gatgataaat ggtgacttca 1560
   cgtcccctct gactcagtac aacagaccag aggatgattg ggcttctgat tatgatcttg 1620
   ttcaggcgat tcaatgtcta cgactgcctg ctaccgtggt tcggaatcgc gcctgtccta 1680
   acgccaagta ccttataaaa cttaacggag ttcactggga ggtagaggtg aggtctggaa 1740
   tggctcctcg ctccctttct cgtgaatgtg tggttggcgt ttgctctgaa ggctgtgtcg 1800
   caccgcctta tccagcagac gggctaccta aacgtgcact cgaggccttg gcgtctgctt 1860
   acagactacc ctccgattgt gttagctctg gtattgctga ctttcttgct aatccacctc 1920
   ctcaggaatt ctggaccctc gacaaaatgt tgacctcccc gtcaccagag cggtccggct 1980
   tctctagttt gtataaatta ctattagagg ttgttccgca aaaatgcggt gccacggaag 2040
   gggctttcat ctatgctgtt gagaggatgt tgaaggattg tccgagctcc aaacaggcca 2100
   tggcccttct ggcaaaaatt aaagttccat cctcaaaggc cccgtctgtg tccctggacg 2160
   agtgtttccc tacggatgtt ttagccgact tcgagccagc atctcaggaa aggccccaaa 2220
   gttccggcgc tgctgttgtc ctgtgttcac cggatgcaaa agagttcgag gaagcagccc 2280
   cggaagaagt tcaagagagt ggccacaagg ccgtccactc tgcactcctt gccgagggtc 2340
   ctaacaatga gcaggtacag gtggttgccg gtgagcaact gaagctcggc ggttgtggtt 2400
   tggcagtcgg gaatgctcat gaaggtgctc tggtctcagc tggtctaatt aacctggtag 2460
   gcgggaattt gtccccctca gaccccatga aagaaaacat gctcaatagc cgggaagacg 2520
   aaccactgga tttgtcccaa ccagcaccag cttccacaac gacccttgtg agagagcaaa 2580
   cacccgacaa cccaggttct gatgccggtg ccctccccgt caccgttcga gaatttgtcc 2640
   cgacggggcc tatactctgt catgttgagc actgcggcac ggagtcgggc gacagcagtt 2700
   cgcctttgga tctatctgat gcgcaaaccc tggaccagcc tttaaatcta tccctggccg 2760
   cttggccagt gagggccacc gcgtctgacc ctggctgggt ccacggtagg cgcgagcctg 2820
   tctttgtaaa gcctcgaaat gctttctctg atggcgattc agcccttcag ttcggggagc 2880
   tttctgaatc cagctctgtc atcgagtttg accggacaaa agatgctccg gtggttgacg 2940
   cccctgtcga cttgacgact tcgaacgagg ccctctctgt agtcgatcct ttcgaatttg 3000
   ccgaactcaa gcgcccgcgt ttctccgcac aagccttaat tgaccgaggc ggtccacttg 3060
   ccgatgtcca tgcaaaaata aagaaccggg tatatgaaca gtgcctccaa gcttgtgagc 3120
   ccggtagtcg tgcaacccca gccaccaggg agtggctcga caaaatgtgg gatagggtgg 3180
   acatgaaaac ttggcgctgc acctcgcagt tccaagctgg tcgcattctt gcgtccctca 3240
   aattcctccc tgacatgatt caagacacac cgcctcctgt tcccaggaag aaccgagcta 3300
   gtgacaatgc cggcctgaag caactggtgg cacagtggga taggaaattg agtgtgaccc 3360
   cccccccaaa accggttggg ccagtgcttg accagatcgt ccctccgcct acggatatcc 3420
   agcaagaaga tgtcaccccc tccgatgggc caccccatgc gccggatttt cctagtcgag 3480
   tgagcacggg cgggagttgg aaaggcctta tgctttccgg cacccgtctc gcggggtcta 3540
   tcagccagcg ccttatgaca tgggtttttg aagttttctc ccacctccca gcttttatgc 3600
   tcacactttt ctcgccgcgg ggctctatgg ctccaggtga ttggttgttt gcaggtgtcg 3660
   ttttacttgc tctcttgctc tgtcgttctt acccgatact cggatgcctt cccttattgg 3720
   gtgtcttttc tggttctttg cggcgtgttc gtctgggtgt ttttggttct tggatggctt 3780
   ttgctgtatt tttattctcg actccatcca acccagtcgg ttcttcttgt gaccacgatt 3840
   cgccggagtg tcatgctgag cttttggctc ttgagcagcg ccaactttgg gaacctgtgc 3900
   gcggccttgt ggtcggcccc tcaggcctct tatgtgtcat tcttggcaag ttactcggtg 3960
   ggtcacgtta tctctggcat gttctcctac gtttatgcat gcttgcagat ttggcccttt 4020
   ctcttgttta tgtggtgtcc caggggcgtt gtcacaagtg ttggggaaag tgtataagga 4080
   cagctcctgc ggaggtggct cttaatgtat ttcctttctc gcgcgccacc cgtgtctctc 4140
   ttgtatcctt gtgtgatcga ttccaaacgc caaaaggggt tgatcctgtg cacttggcaa 4200
   cgggttggcg cgggtgctgg cgtggtgaga gccccatcca tcaaccacac caaaagccca 4260
   tagcttatgc caatttggat gaaaagaaaa tgtctgccca aacggtggtt gctgtcccat 4320
   acgatcccag tcaggctatc aaatgcctga aagttctgca ggcgggaggg gccatcgtgg 4380
   accagcctac acctgaggtc gttcgtgtgt ccgagatccc cttctcagcc ccatttttcc 4440
   caaaagttcc agtcaaccca gattgcaggg ttgtggtaga ttcggacact tttgtggctg 4500
   cggttcgctg cggttactcg acagcacaac tggttctggg ccggggcaac tttgccaagt 4560
   taaatcagac cccccccagg aactctatct ccaccaaaac gactggtggg gcctcttaca 4620
   cccttgctgt ggctcaagtg tctgcgtgga ctcttgttca tttcatcctc ggtctttggt 4680
   tcacatcacc tcaagtgtgt ggccgaggaa ccgctgaccc atggtgttca aatccttttt 4740
   catatcctac ctatggcccc ggagttgtgt gctcctctcg actttgtgtg tctgccgacg 4800
   gggtcaccct gccattgttc tcagccgtgg cacaactctc cggtagagag gtggggattt 4860
   ttattttggt gctcgtctcc ttgactgctt tggcccaccg catggctctt aaggcagaca 4920
   tgttagtggt cttttcggct ttttgtgctt acgcctggcc catgagctcc tggttaatct 4980
   gcttctttcc tatactcttg aagtgggtta cccttcaccc tcttactatg ctttgggtgc 5040
   actcattctt ggtgttttgt ctgccagcag ccggcatcct ctcactaggg ataactggcc 5100
   ttctttgggc aattggccgc tttacccagg ttgccggaat tattacacct tatgacatcc 5160
   accagtacac ctctgggcca cgtggtgcag ctgctgtggc cacagcccca gaaggcactt 5220
   atatggccgc cgtccggaga gctgctttaa ctgggcgaac tttaatcttc accccgtctg 5280
   cagttggatc ccttctcgaa ggtgctttca ggactcataa accctgcctt aacaccgtga 5340
   atgttgtagg ctcttccctt ggttccggag gggttttcac cattgatggc agaagaactg 5400
   tcgtcactgc tgcccatgtg ttgaacggcg acacagctag agtcaccggc gactcctaca 5460
   accgcatgca cactttcaag accaatggtg attatgcctg gtcccatgct gatgactggc 5520
   agggcgttgc ccctgtggtc aaggttgcga aggggtaccg cggtcgtgcc tactggcaaa 5580
   catcaactgg tgtcgaaccc ggtatcattg gggaagggtt cgccttctgt tttactaact 5640
   gcggcgattc ggggtcaccc gtcatctcag aatctggtga tcttattgga atccacaccg 5700
   gttcaaacaa acttggttct ggtcttgtga caacccctga aggggagacc tgcaccatca 5760
   aagaaaccaa gctctctgac ctttccagac attttgcagg cccaagcgtt cctcttgggg 5820
   acattaaatt gagtccggcc atcatccctg atgtaacatc cattccgagt gacttggcat 5880
   cgctcctagc ctccgtccct gtagtggaag gcggcctctc gaccgttcaa cttttgtgtg 5940
   tctttttcct tctctggcgc atgatgggcc atgcctggac acccattgtt gccgtgggct 6000
   tctttttgct gaatgaaatt cttccagcag ttttggtccg agccgtgttt tcttttgcac 6060
   tctttgtgct tgcatgggcc accccctggt ctgcacaggt gttgatgatt agactcctca 6120
   cggcatctct caaccgcaac aagctttctc tggcgttcta cgcactcggg ggtgtcgtcg 6180
   gtttggcagc tgaaatcggg acttttgctg gcagattgtc tgaattgtct caagctcttt 6240
   cgacatactg cttcttacct agggtccttg ctatgaccag ttgtgttccc accatcatca 6300
   ttggtggact ccataccctc ggtgtgattc tgtggttatt caaataccgg tgcctccaca 6360
   acatgctggt tggtgatggg agtttttcaa gcgccttctt cctacggtat tttgcagagg 6420
   gtaatctcag aaaaggtgtt tcacagtcct gtggcatgaa taacgagtcc ctaacggctg 6480
   ctttagcttg caagttgtca caggctgacc ttgatttttt gtccagctta acgaacttca 6540
   agtgctttgt atctgcttca aacatgaaaa atgctgccgg ccagtacatt gaagcagcgt 6600
   atgccaaggc cctgcgccaa gagttggcct ctctagttca gattgacaaa atgaaaggag 6660
   ttttgtccaa gctcgaggcc tttgctgaaa cagccacccc gtcccttgac ataggtgacg 6720
   tgattgttct gcttgggcaa catcctcacg gatccatcct cgatattaat gtggggactg 6780
   aaaggaaaac tgtgtccgtg caagagaccc ggagcctagg cggctccaaa ttcagtgttt 6840
   gtactgtcgt gtccaacaca cccgtggacg ccttgaccgg catcccactc cagacaccaa 6900
   cccctctttt tgagaatggt ccgcgtcatc gcagcgagga agacgatctt aaagtcgaga 6960
   ggatgaagaa acactgtgta tccctcggct tccacaacat caatggcaaa gtttactgca 7020
   aaatttggga caagtctacc ggtgacacct tttacacgga tgattcccgg tacacccaag 7080
   accatgcttt tcaggacagg tcagccgact acagagacag ggactatgag ggtgtgcaaa 7140
   ccacccccca acagggattt gatccaaagt ctgaaacccc tgttggcact gttgtgatcg 7200
   gcggtattac gtataacagg tatctgatca aaggtaagga ggttctggtc cccaagcctg 7260
   acaactgcct tgaagctgcc aagctgtccc ttgagcaagc tctcgctggg atgggccaaa 7320
   cttgcgacct tacagctgcc gaggtggaaa agctaaagcg catcattagt caactccaag 7380
   gtttgaccac tgaacaggct ttaaactgtt agccgccagc ggcttgaccc gctgtggccg 7440
   cggcggccta gttgtgactg aaacggcggt aaaaattata aaataccaca gcagaacttt 7500
   caccttaggc cctttagacc taaaagtcac ttccgaggtg gaggtaaaga aatcaactga 7560
   gcagggccac gctgttgtgg caaacttatg ttccggtgtc atcttgatga gacctcaccc 7620
   accgtccctt gtcgacgttc ttctgaaacc cggacttgac acaatacccg gcattcaacc 7680
   agggcatggg gccgggaata tgggcgtgga cggttctatt tgggattttg aaaccgcacc 7740
   cacaaaggca gaactcgagt tatccaagca aataatccaa gcatgtgaag ttaggcgcgg 7800
   ggacgccccg aacctccaac tcccttacaa gctctatcct gttagggggg atcctgagcg 7860
   gcataaaggc cgccttatca ataccaggtt tggagattta ccttacaaaa ctcctcaaga 7920
   caccaagtcc gcaatccacg cggcttgttg cctgcacccc aacggggccc ccgtgtctga 7980
   tggtaaatcc acactaggta ccactcttca acatggtttc gagctttatg tccctactgt 8040
   gccctatagt gtcatggagt accttgattc acgccctgac acccctttta tgtgtactaa 8100
   acatggcact tccaaggctg ctgcagagga cctccaaaaa tacgacctat ccacccaagg 8160
   atttgtcctg cctggggtcc tacgcctagt acgcagattc atctttggcc atattggtaa 8220
   ggcgccgcca ttgttcctcc catcaaccta tcccgccaag aactctatgg cagggatcaa 8280
   tggccagagg ttcccaacaa aggacgttca gagcatacct gaaattgatg aaatgtgtgc 8340
   ccgcgctgtc aaggagaatt ggcaaactgt gacaccttgc accctcaaga aacagtactg 8400
   ttccaagccc aaaaccagga ccatcctggg caccaacaac tttattgcct tggctcacag 8460
   atcggcgctc agtggtgtca cccaggcatt catgaagaag gcttggaagt ccccaattgc 8520
   cttggggaaa aacaaattca aggagctgca ttgcactgtc gccggcaggt gtcttgaggc 8580
   cgacttggcc tcctgtgacc gcagcacccc cgccattgta agatggtttg ttgccaacct 8640
   cctgtatgaa cttgcaggat gtgaagagta cttgcctagc tatgtgctta attgctgcca 8700
   tgacctcgtg gcaacacagg atggtgcctt cacaaaacgc ggtggcctgt cgtccgggga 8760
   ccccgtcacc agtgtgtcca acaccgtata ttcactggta atttatgccc agcacatggt 8820
   attgtcggcc ttgaaaatgg gtcatgaaat tggtcttaag ttcctcgagg aacagctcaa 8880
   gttcgaggac ctccttgaaa ttcagcctat gttggtatac tctgatgatc ttgtcttgta 8940
   cgctgaaaga cccacatttc ccaattacca ctggtgggtc gagcaccttg acctgatgct 9000
   gggtttcaga acggacccaa agaaaaccgt cataactgat aaacccagct tcctcggctg 9060
   cagaattgag gcagggcgac agctagtccc caatcgcgac cgcatcctgg ctgctcttgc 9120
   atatcacatg aaggcgcaga acgcctcaga gtattatgcg tctgctgccg caatcctgat 9180
   ggattcatgt gcttgcattg accatgaccc tgagtggtat gaggacctca tctgcggtat 9240
   tgcccggtgc gcccgccagg atggttatag cttcccaggt ccggcatttt tcatgtccat 9300
   gtgggagaag ctgagaagtc ataatgaagg gaagaaattc cgccactgcg gcatctgcga 9360
   cgccaaagcc gactatgcgt ccgcctgtgg gcttgatttg tgtttgttcc attcgcactt 9420
   tcatcaacac tgccctgtca ctctgagctg cggtcaccat gccggttcaa aggaatgttc 9480
   gcagtgtcag tcacctgttg gggctggcag atcccctctt gatgccgtgc taaaacaaat 9540
   tccatacaaa cctcctcgta ctgtcatcat gaaggtgggt aataaaacaa cggccctcga 9600
   tccggggagg taccagtccc gtcgaggtct cgttgcagtc aagaggggta ttgcaggcaa 9660
   tgaagttgat ctttctgatg gggactacca agtggtgcct cttttgccga cttgcaaaga 9720
   cataaacatg gtgaaggtgg cttgcaatgt actactcagc aagttcatag tagggccacc 9780
   aggttccgga aagaccacct ggctactgag tcaagtccag gacgatgatg tcatttacac 9840
   acccacccat cagactatgt ttgatatagt cagtgctctc aaagtttgca ggtattccat 9900
   tccaggagcc tcaggactcc ctttcccacc acctgccagg tccgggccgt gggttaggct 9960
   tattgccagc gggcacgtcc ctggccgagt atcatacctc gatgaggctg gatattgtaa 10020
   tcatctggac attcttagac tgctttccaa aacacccctt gtgtgtttgg gtgaccttca 10080
   gcaacttcac cctgtcggct ttgattccta ctgttatgtg ttcgatcaga tgcctcagaa 10140
   gcagctgacc actatttaca gatttggccc taacatctgc gcagccatcc agccttgtta 10200
   cagggagaaa cttgaatcta aggctaggaa cactagggtg gtttttacca cccggcctgt 10260
   ggcctttggt caggtgctga caccatacca taaagatcgc atcggctctg cgataaccat 10320
   agattcatcc cagggggcca cctttgatat tgtgacattg catctaccat cgccaaagtc 10380
   cctaaataaa tcccgagcac ttgtagccat cactcgggca agacacgggt tgttcattta 10440
   tgaccctcat aaccagctcc aggagttttt caacttaacc cctgagcgca ctgattgtaa 10500
   ccttgtgttc agccgtgggg atgagctggt agttctgaat gcggataatg cagtcacaac 10560
   tgtagcgaag gcccttgaga caggtccatc tcgatttcga gtatcagacc cgaggtgcaa 10620
   gtctctctta gccgcttgtt cggccagtct ggaagggagc tgtatgccac taccgcaagt 10680
   ggcacataac ctggggtttt acttttcccc ggacagtcca acatttgcac ctctgccaaa 10740
   agagttggcg ccacattggc cagtggttac ccaccagaat aatcgggcgt ggcctgatcg 10800
   acttgtcgct agtatgcgcc caattgatgc ccgctacagc aagccaatgg tcggtgcagg 10860
   gtatgtggtc gggccgtcca cctttcttgg tactcctggt gtggtgtcat actatctcac 10920
   actatacatc aggggtgagc cccaggcctt gccagaaaca ctcgtttcaa cagggcgtat 10980
   agccacagat tgtcgggagt atctcgacgc ggctgaggaa gaggcagcaa aagaactccc 11040
   ccacgcattc attggcgatg tcaaaggtac cacggttggg gggtgtcatc acattacatc 11100
   aaaataccta cctaggtccc tgcctaagga ctctgttgcc gtagttggag taagttcgcc 11160
   cggcagggct gctaaagccg tgtgcactct caccgatgtg tacctccccg aactccggcc 11220
   atatctgcaa cctgagacgg catcaaaatg ctggaaactc aaattagact tcagggacgt 11280
   ccgactaatg gtctggaaag gagccaccgc ctatttccag ttggaagggc ttacatggtc 11340
   ggcgctgccc gactatgcca ggtttattca gctgcccaag gatgccgttg tatacattga 11400
   tccgtgtata ggaccggcaa cagccaaccg taaggtcgtg cgaaccacag actggcgggc 11460
   cgacctggca gtgacaccgt atgattacgg tgcccagaac attttgacaa cagcctggtt 11520
   cgaggacctc gggccgcagt ggaagatttt ggggttgcag ccctttaggc gagcatttgg 11580
   ctttgaaaac actgaggatt gggcaatcct tgcacgccgt atgaatgacg gcaaggacta 11640
   cactgactat aactggaact gtgttcgaga acgcccacac gccatctacg ggcgtgctcg 11700
   tgaccatacg tatcattttg cccctggcac agaattgcag gtagagctag gtaaaccccg 11760
   gctgccgcct gggcaagtgc cgtgaattcg gggtgatgca atggggtcac tgtggagtaa 11820
   aatcagccag ctgttcgtgg acgccttcac tgagttcctt gttagtgtgg ttgatattgc 11880
   cattttcctt gccatactgt ttgggttcac cgtcgcagga tggttactgg tctttcttct 11940
   cagagtggtt tgctccgcgc ttctccgttc gcgctctgcc attcactctc ccgaactatc 12000
   gaaggtccta tgaaggcttg ttgcccaact gcagaccgga tgtcccacaa tttgcagtca 12060
   agcacccatt gggtatgttt tggcacatgc gagtttccca cttgattgat gagatggtct 12120
   ctcgtcgcat ttaccagacc atggaacatt caggtcaagc ggcctggaag caggtggttg 12180
   gtgaggccac tctcacgaag ctgtcagggc tcgatatagt tactcatttc caacacctgg 12240
   ccgcagtgga ggcggattct tgccgctttc tcagctcacg actcgtgatg ctaaaaaatc 12300
   ttgccgttgg caatgtgagc ctacagtaca acaccacgtt ggaccgcgtt gagctcatct 12360
   tccccacgcc aggtacgagg cccaagttga ccgatttcag acaatggctc atcagtgtgc 12420
   acgcttccat tttttcctct gtggcttcat ctgttacctt gttcatagtg ctttggcttc 12480
   gaattccagc tctacgctat gtttttggtt tccattggcc cacggcaaca catcattcga 12540
   gctgaccatc aactacacca tatgcatgcc ctgttctacc agtcaagcgg ctcgccaaag 12600
   gctcgagccc ggtcgtaaca tgtggtgcaa aatagggcat gacaggtgtg aggagcgtga 12660
   ccatgatgag ttgttaatgt ccatcccgtc cgggtacgac aacctcaaac ttgagggtta 12720
   ttatgcttgg ctggcttttt tgtccttttc ctacgcggcc caattccatc cggagttgtt 12780
   cgggataggg aatgtgtcgc gcgtcttcgt ggacaagcga caccagttca tttgtgccga 12840
   gcatgatgga cacaattcaa ccgtatctac cggacacaac atctccgcat tatatgcggc 12900
   atattaccac caccaaatag acgggggcaa ttggttccat ttggaatggc tgcggccact 12960
   cttttcttcc tggctggtgc tcaacatatc atggtttctg aggcgttcgc ctgtaagccc 13020
   tgtttctcga cgcatctatc agatattgag accaacacga ccgcggctgc cggtttcatg 13080
   gtccttcagg acatcaattg tttccgacct cacggggtct cagcagcgca agagaaaatt 13140
   tccttcggaa agtcgtccca atgtcgtgaa gccgtcggta ctccccagta catcacgata 13200
   acggctaacg tgaccgacga atcatacttg tacaacgcgg acctgctgat gctttctgcg 13260
   tgccttttct acgcctcaga aatgagcgag aaaggcttca aagtcatctt tgggaatgtc 13320
   tctggcgttg tttctgcttg tgtcaatttc acagattatg tggcccatgt gacccaacat 13380
   acccagcagc atcatctggt aattgatcac attcggttgc tgcatttcct gacaccatct 13440
   gcaatgaggt gggctacaac cattgcttgt ttgttcgcca ttctcttggc aatatgagat 13500
   gttctcacaa attggggcgt ttcttgactc cgcactcttg cttctggtgg ctttttttgc 13560
   tgtgtaccgg cttgtcctgg tcctttgccg atggcaacgg cgacagctcg acataccaat 13620
   acatatataa cttgacgata tgcgagctga atgggaccga ctggttgtcc agccattttg 13680
   gttgggcagt cgagaccttt gtgctttacc cggttgccac tcatatcctc tcactgggtt 13740
   ttctcacaac aagccatttt tttgacgcgc tcggtctcgg cgctgtatcc actgcaggat 13800
   ttgttggcgg gcggtacgta ctctgcagcg tctacggcgc ttgtgctttc gcagcgttcg 13860
   tatgttttgt catccgtgct gctaaaaatt gcatggcctg ccgctatgcc cgtacccggt 13920
   ttaccaactt cattgtggac gaccggggga gagttcatcg atggaagtct ccaatagtgg 13980
   tagaaaaatt gggcaaagcc gaagtcgatg gcaacctcgt caccatcaaa catgtcgtcc 14040
   tcgaaggggt taaagctcaa cccttgacga ggacttcggc tgagcaatgg gaggcctaga 14100
   cgatttttgc aacgatccta tcgccgcaca aaagctcgtg ctagccttta gcatcacata 14160
   cacacctata atgatatacg cccttaaggt gtcacgcggc cgactcctgg ggctgttgca 14220
   catcctaata tttctgaact gttcctttac attcggatac atgacatatg tgcattttca 14280
   atccaccaac cgtgtcgcac ttaccctggg ggctgttgtc gcccttctgt ggggtgttta 14340
   cagcttcaca gagtcatgga agtttatcac ttccagatgc agattgtgtt gccttggccg 14400
   gcgatacatt ctggcccctg cccatcacgt agaaagtgct gcaggtctcc attcaatctc 14460
   agcgtctggt aaccgagcat acgctgtgag aaagcccgga ctaacatcag tgaacggcac 14520
   tctagtacca ggacttcgga gcctcgtgct gggcggcaaa cgagctgtta aacgaggagt 14580
   ggttaacctc gtcaagtatg gccggtaaaa accagagcca gaagaaaaag aaaagtacag 14640
   ctccgatggg gaatggccag ccagtcaatc aactgtgcca gttgctgggt gcaatgataa 14700
   agtcccagcg ccagcaacct aggggaggac aggccaaaaa gaaaaagcct gagaagccac 14760
   attttcccct ggctgctgaa gatgacatcc ggcaccacct cacccagact gaacgctccc 14820
   tctgcttgca atcgatccag acggctttca atcaaggcgc aggaactgcg tcgctttcat 14880
   ccagcgggaa ggtcagtttt caggttgagt ttatgctgcc ggttgctcat acagtgcgcc 14940
   tgattcgcgt gacttctaca tccgccagtc agggtgcaag ttaatttgac agtcaggtga 15000
   atggccgcga ttggcgtgtg gcctctgagt cacctattca attagggcga tcacatgggg 15060
   gtcatactta atcaggcagg aaccatgtga ccgaaattaa aaaaaaaaaa a 15111
<210> 21
   <211> 15411
   <212> DNA
   <213> Porcine reproductive and respiratory syndrome virus
<400> 21
   atgacgtata ggtgttggct ctatgccttg gcatttgtat tgtcaggagc tgtgaccatt 60
   ggcacagccc aaaacttgct gcacagaaac acccttctgt gatagcctcc ttcaggggag 120
   cttagggttt gtccctagca ccttgcttcc ggagttgcac tgctttacgg tctctccacc 180
   cctttaacca tgtctgggat acttgatcgg tgcacgtgta cccccaatgc cagggtgttt 240
   atggcggagg gccaagtcta ctgcacacga tgcctcagtg cacggtctct ccttcccctg 300
   aacctccaag tttctgagct cggggtgcta ggcctattct acaggcccga agagccactc 360
   cggtggacgt tgccacgtgc attccccact gttgagtgct cccccgccgg ggcctgctgg 420
   ctttctgcaa tctttccaat cgcacgaatg accagtggaa acctgaactt ccaacaaaga 480
   atggtacggg tcgcagctga gctttacaga gccggccagc tcacccctgc agtcttgaag 540
   gctctacaag tttatgaacg gggttgccgc tggtacccca ttgttggacc tgtccctgga 600
   gtggccgttt tcgccaattc cctacatgtg agtgataaac ctttcccggg agcaactcac 660
   gtgttgacca acctgccgct cccgcagaga cccaagcctg aagacttttg cccctttgag 720
   tgtgctatgg ctactgtcta tgacattggt catgacgccg tcatgtatgt ggccgaaagg 780
   aaagtctcct gggcccctcg tggcggggat gaagtgaaat ttgaagctgt ccccggggag 840
   ttgaagttga ttgcgaaccg gctccgcacc tccttcccgc cccaccacac agtggacatg 900
   tctaagttcg ccttcacagc ccctgggtgt ggtgtttcta tgcgggtcga acgccaacac 960
   ggctgccttc ccgctgacac tgtccctgaa ggcaactgct ggtggagctt gtttgacttg 1020
   cttccactgg aagttcagaa caaagaaatt cgccatgcta accaatttgg ctaccagacc 1080
   aagcatggtg tctctggcaa gtacctacag cggaggctgc aagttaatgg tctccgagca 1140
   gtaactgacc taaacggacc tatcgtcgta cagtacttct ccgttaagga gagttggatc 1200
   cgccatttga aactggcggg agaacccagc tactctgggt ttgaggacct cctcagaata 1260
   agggttgagc ctaacacgtc gccattggct gacaaggaag aaaaaatttt ccggtttggc 1320
   agtcacaagt ggtacggcgc tggaaagaga gcaagaaaag cacgctcttg tgcgactgct 1380
   acagtcgctg gccgcgcttt gtccgttcgt gaaacccggc aggccaagga gcacgaggtt 1440
   gccggcgcca acaaggctga gcacctcaaa cactactccc cgcctgccga agggaattgt 1500
   ggttggcact gcatttccgc catcgccaac cggatggtga attccaaatt tgaaaccacc 1560
   cttcccgaaa gagtgagacc tccagatgac tgggctactg acgaggatct tgtgaatgcc 1620
   atccaaatcc tcagactccc tgcggcctta gacaggaacg gtgcttgtac tagcgccaag 1680
   tacgtactta agctggaagg tgagcattgg actgtcactg tgacccctgg gatgtcccct 1740
   tctttgctcc ctcttgaatg tgttcagggc tgttgtgggc acaagggcgg tcttggttcc 1800
   ccagatgcag tcgaggtctc cggatttgac cctgcctgcc ttgaccggct ggctgaggtg 1860
   atgcacctgc ctagcagtgc tatcccagcc gctctggccg aaatgtctgg cgattccgat 1920
   cgttcggctt ctccggtcac caccgtgtgg actgtttcgc agttctttgc ccgtcacagc 1980
   ggagggaatc accctgacca agtgcgctta gggaaaatta tcagcctttg tcaggtgatt 2040
   gaggactgct gctgttccca gaacaaaacc aaccgggtca ccccggagga ggtcgcagca 2100
   aagattgacc tgtacctccg tggtgcaaca aatcttgaag aatgcttggc caggcttgag 2160
   aaagcgcgcc cgccacgcgt aatcgacacc tcctttgatt gggatgttgt gctccctggg 2220
   gttgaggcgg caacccagac gatcaagctg ccccaggtca accagtgtcg tgctctggtc 2280
   cctgttgtga ctcaaaagtc cttggacaac aactcggtcc ccctgaccgc cttttcactg 2340
   gctaactact actaccgtgc gcaaggtgac gaagttcgtc accgtgaaag actaaccgcc 2400
   gtgctctcca agttggaaaa ggttgttcga gaagaatatg ggctcatgcc aaccgagcct 2460
   ggtccacggc ccacactgcc acgcgggctc gacgaactca aagaccagat ggaggaggac 2520
   ttgctgaaac tggctaacgc ccagacgact tcggacatga tggcctgggc agtcgagcag 2580
   gttgacctaa aaacttgggt caagaactac ccgcggtgga caccaccacc ccctccgcca 2640
   aaagttcagc ctcgaaaaac gaagcctgtc aagagcttgc cggagagaaa gcctgtcccc 2700
   gccccgcgca ggaaggttgg gtccgattgt ggcagcccgg tttcattagg cggcgatgtc 2760
   cctaacagtt gggaagattt ggctgttagt agcccctttg atctcccgac cccacctgag 2820
   ccggcaacac cttcaagtga gctggtgatt gtgtcctcac cgcaatgcat cttcaggccg 2880
   gcgacaccct tgagtgagcc ggctccaatt cccgcacctc gcggaactgt gtctcgaccg 2940
   gtgacaccct tgagtgagcc gatccctgtg cccgcaccgc ggcgtaagtt tcagcaggtg 3000
   aaaagattga gttcggcggc ggcaatccca ccgtaccagg acgagcccct ggatttgtct 3060
   gcttcctcac agactgaata tgaggcctct cccccagcac cgccgcagag cgggggcgtt 3120
   ctgggagtag aggggcatga agctgaggaa accctgagtg aaatctcgga catgtcgggt 3180
   aacattaaac ctgcgtccgt gtcatcaagc agctccttgt ccagcgtgag aatcacacgc 3240
   ccaaaatact cagctcaagc catcatcgac tcgggcgggc cctgcagtgg gcatctccaa 3300
   gaggtaaagg aaacatgcct tagtgtcatg cgcgaggcat gtgatgcgac taagcttgat 3360
   gaccctgcta cgcaggaatg gctttctcgc atgtgggatc gggtggacat gctgacttgg 3420
   cgcaacacgt ctgtttacca ggcgatttgc accttagatg gcaggttaaa gttcctccca 3480
   aaaatgatac tcgagacacc gccgccctat ccgtgtgagt ttgtgatgat gcctcacacg 3540
   cctgcacctt ccgtaggtgc ggagagcgac cttaccattg gctcagttgc tactgaagat 3600
   gttccacgca tcctcgagaa aatagaaaat gtcggcgaga tggccaacca gggacccttg 3660
   gccttctccg aggataaacc ggtagatgac caacttgtca acgacccccg gatatcgtcg 3720
   cggaggcctg acgagagcac atcagctccg tccgcaggca caggtggcgc cggctctttt 3780
   accgatttgc cgccttcaga tggcgcggat gcggacgggg gggggccgtt tcggacggta 3840
   aaaagaaaag ctgaaaggct ctttgaccaa ctgagccgtc aggtttttga cctcgtctcc 3900
   catctccctg ttttcttctc acgccttttc taccctggcg gtggttattc tccgggtgat 3960
   tggggttttg cagcttttac tctattgtgc ctctttttat gttacagtta cccagccttt 4020
   ggtattgctc ccctcttggg tgtgttttct gggtcttctc ggcgcgttcg aatgggggtt 4080
   tttggctgct ggttggcttt tgctgttggt ctgttcaagc ctgtgtccga cccagtcggc 4140
   gctgcttgtg agtttgactc gccagagtgt agaaacatcc ttcattcttt tgagcttctc 4200
   aaaccttggg accctgttcg cagccttgtt gtgggccccg tcggtctcgg tcttgccatt 4260
   cttggcaggt tactgggcgg ggcacgctgc atctggcact ttttgcttag gcttggcatt 4320
   gttgcagact gtatcttggc tggagcttac gtgctttctc aaggtaggtg taaaaagtgc 4380
   tggggatctt gtataagaac tgctcctaat gaggtcgctt ttaacgtgtt tcctttcaca 4440
   cgtgcgacca ggtcgtcact tatcgacctg tgcgatcggt tttgtgcgcc aaaaggaatg 4500
   gaccccattt ttctcgccac tgggtggcgc gggtgctggg ccggccgaag ccccattgag 4560
   caaccctctg aaaaacccat cgcgtttgcc caattggatg aaaagaagat tacggctagg 4620
   actgtggtcg cccagcctta tgaccccaac caagccgtaa agtgcttgcg ggtattgcag 4680
   tcgggtgggg cgatggtggc taaggcggtc ccaaaagtgg tcaaggtttc cgctgttcca 4740
   ttccgagccc ccttctttcc cactggagtg aaagttgacc ctgattgcag ggtcgtggtt 4800
   gaccctgaca ctttcactgc agctctccgg tctggctact ccaccacaaa cctcgtcctt 4860
   ggtgtagggg actttgccca gctgaatgga ttaaaaatca ggcaaatttc caagccttca 4920
   gggggaggcc cacatctcat ggctgccctg catgttgcct gctcgatggc tctgcacatg 4980
   cttgctggga tttatgtgac tgcggtgggt tcttgcggca ccggcaccaa cgacccgtgg 5040
   tgcgctaacc cgtttgccgt ccctggctac ggacctggct ctctctgcac gtccaggttg 5100
   tgcatttccc aacacggcct taccctgccc ttgacagcac ttgtggcggg attcggtatt 5160
   caagaaattg ccttggtcgt tttgattttt gtttccatcg gaggcatggc tcataggttg 5220
   agctgtaagg ctgacatgct gtgtgttttg cttgcaattg ccagctatgt ttgggtacct 5280
   cttacctggt tgctttgtgt gtttccttgc tggttgcgct gtttttcttt gcaccccctc 5340
   accatcctat ggttggtgtt tttcttgatt tctgtgaata tgccttcagg aatcttggcc 5400
   atggtgttgt tggtttctct ttggcttctt ggtcgttata ctaatgttgc tggccttgtc 5460
   accccctacg acattcatca ttacaccagt ggcccccgcg gtgttgccgc cttggctacc 5520
   gcaccagatg ggacctactt ggccgctgtc cgccgcgctg cgttgactgg ccgcaccatg 5580
   ctgtttaccc cgtcccagct tgggtctctt cttgagggtg ctttcagaac tcgaaagccc 5640
   tcactgaaca ccgtcaatgt gatcgggtcc tccatgggct ctggcggggt gtttaccatc 5700
   gacgggaaag tcaagtgcgt aactgccgca catgtcctta cgggcaattc agctcgggtt 5760
   tccggggtcg gcttcaatca aatgcttgac tttgacgtaa agggagattt cgctatagct 5820
   gattgcccga attggcaagg ggctgccccc aagacccaat tctgcacgga tggatggact 5880
   ggccgtgcct attggctaac atcctctggc gtcgaacccg gcgtcattgg aaaaggattc 5940
   gccttctgct tcaccgcatg tggcgattcc gggtccccag tgatcaccga ggccggtgag 6000
   cttgtcggcg ttcacacggg atcgaataaa caaggggggg gcattgttac gcgcccctca 6060
   ggccagtttt gtaatgtggc acccatcaag ctaagcgaat taagtgaatt ctttgctggg 6120
   cctaaggtcc cgctcggtga tgtgaaggtc ggcagccaca taattaaaga cataagcgag 6180
   gtgccttcag atctttgtgc cttgcttgct gccaaacctg aactggaagg aggcctctcc 6240
   accgtccaac ttctttgtgt gttttttctc ctgtggagaa tgatgggaca tgcctggacg 6300
   cccttggttg ctgtgagttt ctttattttg aatgaggttc tcccagccgt cctggtccgg 6360
   agtgttttct cctttggaat gtttgtgcta tcctggctca cgccatggtc tgcgcaagtt 6420
   ctgatgatca ggcttctgac agcagctctt aacaggaaca gatggtcact tgcctttttc 6480
   agcctcggtg cagtgaccgg ttttgtcgca gatcttgcgg ccactcaggg gcatccgttg 6540
   caggcagtga tgaatttgag cacctatgca ttcctgcctc ggatgatggt tgtgacctca 6600
   ccagtcccag tgatcacgtg tggtgtcgtg cacctacttg ccatcatttt gtacttgttt 6660
   aagtaccgtg gcccgcacca tatccttgtt ggcgatggag tgttctctgc ggctttcttc 6720
   ttgagatact ttgccgaggg aaagttgagg gaaggggtgt cgcaatcctg cggaatgaat 6780
   catgagtctc tgactggtgc cctcgctatg agactcaatg acgaggactt ggatttcctt 6840
   atgaaatgga ctgattttaa gtgctttgtt tctgcgtcca acatgaggaa tgcagcgggt 6900
   caatttatcg aggctgccta tgctaaagca cttagagtag aactggccca gttggtgcag 6960
   gttgataaag ttcgaggtac tttggccaaa cttgaagctt ttgctgatac cgtggcacct 7020
   caactctcgc ccggtgacat tgttgtcgct ctcggccaca cgcctgttgg cagtatcttc 7080
   gacctaaagg ttggtagcac caagcatacc ctccaagcca ttgagaccag agtccttgct 7140
   gggtccaaaa tgaccgtggc gcgcgtcgtc gacccgaccc ccacgccccc acccgcaccc 7200
   gtgcccatcc ccctcccacc gaaagttctg gagaatggcc ccaacgcttg gggggatgag 7260
   gaccgtttga ataagaagaa gaggcgcagg atggaagccc tcggcatcta tgttatgggc 7320
   gggaaaaagt accagaaatt ttgggacaag aattccggtg atgtgtttta tgaggaggtc 7380
   cataataaca cagatgagtg ggagtgtctc agagttggcg accctgccga ctttgaccct 7440
   gagaagggaa ctctgtgtgg acatgtcacc attgaaaaca aggcttacca tgtttacacc 7500
   tccccatctg gtaagaagtt cttggtcccc gtcaacccag agaatggaag agttcaatgg 7560
   gaagctgcaa agctttccgt ggagcaggcc ctaggtatga tgaatgtcga cggcgaactg 7620
   actgccaaag aactggagaa actgaaaaga ataattgaca aactccaggg cctgactaag 7680
   gagcagtgtt taaactgcta gccgccagcg acttgacccg ctgtggtcgc ggcggcttgg 7740
   ttgttactga aacagcggta aaaatagtca aatttcacaa ccggaccttc accctgggac 7800
   ctgtgaattt aaaagtggcc agtgaggttg agctaaaaga cgcggttgag cacaaccaac 7860
   acccggttgc gagaccgatc gatggtggag ttgtgctcct gcgttccgcg gttccttcgc 7920
   ttatagacgt cttgatctcc ggtgctgatg catctcccaa gttacttgcc catcacgggc 7980
   cgggaaacac tgggatcgat ggcacgctct gggattttga gtccgaagcc actaaagagg 8040
   aagtcgcact cagtgcgcaa ataatacagg cttgtgacat taggcgcggc gacgctcctg 8100
   aaattggtct cccttacaag ctgtaccctg ttaggggtaa ccctgagcgg gtgaaaggag 8160
   ttctgcagaa tacaaggttt ggagacatac cttacaaaac ccccagtgac actggaagcc 8220
   cagtgcacgc ggctgcctgc cttacgccca acgccactcc ggtgactgat gggcgctccg 8280
   tcttggccac gaccatgccc cccgggtttg agttatatgt accgaccata ccagcgtctg 8340
   tccttgatta ccttgactct aggcctgact gccctaaaca gctgacagag cacggctgcg 8400
   aagatgccgc actgaaagac ctctctaaat atgacttgtc cacccaaggc tttgttttac 8460
   ctggagttct tcgccttgtg cggaaatacc tgtttgccca tgtaggtaag tgcccacccg 8520
   ttcatcggcc ttctacttac cctgctaaga attctatggc tggaataaat gggaacaggt 8580
   tcccaaccaa ggacattcag agcgtccctg aaatcgacgt tctgtgcgca caggctgtgc 8640
   gagaaaactg gcaaactgtc accccttgta ctcttaagaa acagtattgc gggaagaaga 8700
   agactaggac catactcggc accaataact tcatcgcact agcccaccga gcagtgttga 8760
   gtggtgttac ccagggcttc atgaaaaagg cgtttaactc gcccatcgcc ctcggaaaga 8820
   acaagtttaa ggagctacag actccggtcc tgggcaggtg ccttgaagct gatctcgcat 8880
   cctgcgatcg atccacgcct gcaattgtcc gctggtttgc cgccaacctt ctttatgaac 8940
   ttgcctgtgc tgaagagcat ctaccgtcgt acgtgctgaa ctgctgccac gacttactgg 9000
   tcacgcagtc cggcgcagtg actaagagag gtggcctgtc gtctggcgac ccgatcacct 9060
   ctgtgtctaa caccatttat agtttggtga tctatgcaca gcatatggtg cttagttact 9120
   tcaaaagtgg tcacccccat ggccttctgt tcttacaaga ccagctaaag tttgaggaca 9180
   tgctcaaggt tcaacccctg atcgtctatt cggacgacct cgtgctgtat gccgagtctc 9240
   ccaccatgcc aaactatcac tggtgggttg aacatctgaa tttgatgctg gggtttcaga 9300
   cggacccaaa gaagacagca ataacagact cgccatcatt tctaggctgt agaataataa 9360
   atgggcgcca gctagtcccc aaccgtgaca ggatcctcgc ggccctcgcc tatcacatga 9420
   aggcgagtaa tgtttctgaa tactatgcct cagcggctgc aatactcatg gacagctgtg 9480
   cttgtttgga gtatgatcct gaatggtttg aagaacttgt agttggaata gcgcagtgcg 9540
   cccgcaagga cggctacagc tttcccggca cgccgttctt catgtccatg tgggaaaaac 9600
   tcaggtccaa ttatgagggg aagaagtcga gagtgtgcgg gtactgcggg gccccggccc 9660
   cgtacgctac tgcctgtggc ctcgacgtct gcatttacca cacccacttc caccagcatt 9720
   gtccagtcac aatctggtgt ggccatccag cgggttctgg ttcttgtagt gagtgcaaat 9780
   cccctgtagg gaaaggcaca agccctttag acgaggtgct ggaacaagtc ccgtataagc 9840
   ccccacggac cgttatcatg catgtggagc agggtctcac cccccttgat ccaggtagat 9900
   accaaactcg ccgcggatta gtctctgtca ggcgtggaat taggggaaat gaagttggac 9960
   taccagacgg tgattatgct agcaccgcct tgctccctac ctgcaaagag atcaacatgg 10020
   tcgctgtcgc ttccaatgta ttgcgcagca ggttcatcat cggcccaccc ggtgctggga 10080
   aaacatactg gctccttcaa caggtccagg atggtgatgt tatttacaca ccaactcacc 10140
   agaccatgct tgacatgatt agggctttgg ggacgtgccg gttcaacgtc ccggcaggca 10200
   caacgctgca attccccgtc ccctcccgca ccggtccgtg ggttcgcatc ctagccggcg 10260
   gttggtgtcc tggcaagaat tccttcctag atgaagcagc gtattgcaat caccttgatg 10320
   ttttgaggct tcttagtaaa actaccctca cctgtctagg agacttcaag caactccacc 10380
   cagtgggttt tgattctcat tgctatgttt ttgacatcat gcctcaaact caactgaaga 10440
   ccatctggag gtttggacag aatatctgtg atgccattca gccagattac agggacaaac 10500
   tcatgtccat ggtcaacaca acccgtgtga cctacgtgga aaaacctgtc aggtatgggc 10560
   aggtcctcac cccctaccac agggaccgag aggacgacgc catcactatt gactccagtc 10620
   aaggcgccac attcgatgtg gttacattgc atttgcccac taaagattca ctcaacaggc 10680
   aaagagccct tgttgctatc accagggcaa gacacgctat ctttgtgtat gacccacaca 10740
   ggcagctgca gggcttgttt gatcttcctg caaaaggcac gcccgtcaac ctcgcagtgc 10800
   actgcgacgg gcagctgatc gtgctggata gaaataacaa agaatgcacg gttgctcagg 10860
   ctctaggcaa cggggataaa tttagggcca cagacaagcg tgttgtagat tctctccgcg 10920
   ccatttgtgc tgatctagaa gggtcgagct ctccgctccc caaggtcgca cacaacttgg 10980
   gattttattt ctcacctgat ttaacacagt ttgctaaact cccagtagaa cttgcacctc 11040
   actggcccgt ggtgtcaacc cagaacaatg aaaagtggcc ggatcggctg gttgccagcc 11100
   ttcgccctat ccataaatac agccgcgcgt gcatcggtgc cggctatatg gtgggccctt 11160
   cggtgtttct aggcactcct ggggtcgtgt catactatct cacaaaattt gttaagggcg 11220
   gggctcaagt gcttccggag acggttttca gcaccggccg aattgaggta gactgccggg 11280
   aatatcttga tgatcgggag cgagaagttg ctgcgtccct cccacacggt ttcattggcg 11340
   acgtcaaagg cactaccgtt ggaggatgtc atcatgtcac ctccagatac ctcccgcgcg 11400
   tccttcccaa ggaatcagtt gcggtagtcg gggtttcaag ccccggaaaa gccgcgaaag 11460
   cattgtgcac actgacagat gtgtacctcc cagatcttga agcctatctc cacccggaga 11520
   cccagtccaa gtgctggaaa atgatgttgg acttcaaaga agttcgacta atggtctgga 11580
   aagacaaaac agcctatttc caacttgaag gtcgctattt cacctggtat cagcttgcca 11640
   gctatgcctc gtacatccgt gttcccgtca actctacggt gtacttggac ccctgcatgg 11700
   gccccgccct ttgcaacagg agagtcgtcg ggtccaccca ctggggggct gacctcgcgg 11760
   tcacccctta tgattacggc gctaaaatta tcctgtctag cgcgtaccat ggtgaaatgc 11820
   cccccggata caaaattctg gcgtgcgcgg agttctcgtt ggatgaccca gttaagtaca 11880
   aacatacctg ggggtttgaa tcggatacag cgtatctgta tgagttcacc ggaaacggtg 11940
   aggactggga ggattacaat gatgcgtttc gtgcgcgcca ggaagggaaa atttataagg 12000
   ccactgccac cagcttgaag ttttattttc ccccgggccc tgtcattgaa ccaactttag 12060
   gcctgaattg aaatgaaatg gggtccatgc aaagcctttt tgacaaaatt ggccaacttt 12120
   ttgtggatgc tttcacggag ttcttggtgt ccattgttga tatcattata tttttggcca 12180
   ttttgtttgg cttcaccatc gccggttggc tggtggtctt ttgcatcaga ttggtttgct 12240
   ccgcgatact ccgtacgcgc cctgccattc actctgagca attacagaag atcttatgag 12300
   gcctttcttt cccagtgcca agtggacatt cccacctggg gaactaaaca tcctttgggg 12360
   atgctttggc accataaggt gtcaaccctg attgatgaaa tggtgtcgcg tcgaatgtac 12420
   cgcatcatgg aaaaagcagg gcaggctgcc tggaaacagg tggtgagcga ggctacgctg 12480
   tctcgcatta gtagtttgga tgtggtggct cattttcagc atctagccgc cattgaagcc 12540
   gagacctgta aatatttggc ctcccggctg cccatgctac acaacctgcg catgacaggg 12600
   tcaaatgtaa ccatagtgta taatagcact ttgaatcagg tgtttgctat ttttccaacc 12660
   cctggttccc ggccaaagct tcatgatttt cagcaatggt taatagctgt acattcctcc 12720
   atattttcct ctgttgcagc ttcttgtact ctttttgttg tgctgtggtt gcgggttcca 12780
   atactacgta ctgtttttgg tttccgctgg ttaggggcaa tttttctttc gaactcacag 12840
   tgaattacac ggtgtgtcca ccttgcctca cccggcaagc agccacagag atctacgaac 12900
   ccggtaggtc tctttggtgc aggatagggt atgaccgatg tggggaggac gatcatgacg 12960
   agctagggtt tatgataccg cctggcctct ccagcgaagg ccacttgact ggtgtttacg 13020
   cctggttggc gttcttgtcc ttcagctaca cggcccagtt ccatcccgag atattcggga 13080
   tagggaatgt gagtcgagtt tatgttgaca tcaaacatca actcatctgc gccgaacatg 13140
   acgggcagaa caccaccttg cctcgtcatg acaacatttc agccgtgttt cagacctatt 13200
   accaacatca agtcgacggc ggcaattggt ttcacctaga atggcttcgt cccttctttt 13260
   cctcgtggtt ggttttaaat gtctcttggt ttctcaggcg ttcgcctgca aaccatgttt 13320
   cagttcgagt cttgcagata ttaagaccaa caccaccgca gcggcaagct ttgctgtcct 13380
   ccaagacatc agttgcctta ggcatcgcga ctcggcctct gaggcgattc gcaaaatccc 13440
   tcagtgccgt acggcgatag ggacacccgt gtatgttacc atcacagcca atgtgacaga 13500
   tgagaattat ttacattctt ctgatctcct catgctttct tcttgccttt tctatgcttc 13560
   tgagatgagt gaaaagggat ttaaggtggt atttggcaat gtgtcaggca tcgtggctgt 13620
   gtgtgtcaat tttaccagct acgtccaaca tgtcaaggag tttacccaac gctccctggt 13680
   ggtcgaccat gtgcggttgc tccatttcat gacacctgag accatgaggt gggcaactgt 13740
   tttagcctgt ctttttgcca ttctgttggc aatttgaatg tttaagtatg ttggagaaat 13800
   gcttgaccgc gggctgttgc tcgcgattgc tttctttgtg gtgtatcgtg ccgttctgtt 13860
   ttgctgtgct cgccaacgcc agcaacgaca gcagctccca tctacagctg atttacaact 13920
   tgacgctatg tgagctgaat ggcacagatt ggctagctaa caaatttgat tgggcagtgg 13980
   agagttttgt catctttccc gttttgactc acattgtctc ctatggtgcc ctcactacca 14040
   gccatttcct tgacacagtc gctttagtca ctgtgtctac cgccgggttt gttcacgggc 14100
   ggtatgtcct aagtagcatc tacgcggtct gtgccctggc tgcgttgact tgcttcgtca 14160
   ttaggtttgc aaagaattgc atgtcctggc gctacgcgtg taccagatat accaactttc 14220
   ttctggacac taagggcaga ctctatcgtt ggcggtcgcc tgtcatcata gagaaaaggg 14280
   gcaaagttga ggtcgaaggt catctgatcg acctcaaaag agttgtgctt gatggttccg 14340
   tggcaacccc tataaccaga gtttcagcgg aacaatgggg tcgtccttag atgacttctg 14400
   tcatgatagc acggctccac aaaaggtgct tttggcgttt tctattacct acacgccagt 14460
   gatgatatat gccctaaagg tgagtcgcgg ccgactgcta gggcttctgc accttttgat 14520
   cttcctgaat tgtgctttca ccttcgggta catgactttc gcgcactttc agagtacaaa 14580
   taaggtcgcg ctcactatgg gagcagtagt tgcactcctt tggggggtgt actcagccat 14640
   agaaacctgg aaattcatca cctccagatg ccgtttgtgc ttgctaggcc gcaagtacat 14700
   tctggcccct gcccaccacg ttgaaagtgc cgcacggttt catccgattg cggcaaatga 14760
   taaccacgca tttgtcgtcc ggcgtcccgg ctccactacg gtcaacggca cattggtgcc 14820
   cgggttaaaa agcctcgtgt tgggtggcag aaaagctgtt aaacagggag tggtaaacct    14880
   tgtcaaatat gccaaataac aacggcaagc agcagaagag aaagaagggg gatggccagc    14940
   cagtcaatca gctgtgccag atgctgggta agatcatcgc tcagcaaaac cagtccagag    15000
   gcaagggacc gggaaagaaa aataagaaga aaaacccgga gaagccccat tttcctctag    15060
   cgactgaaga tgatgtcaga catcacttta cccctagtga gcggcaattg tgtctgtcgt    15120
   caatccagac cgcctttaat caaggcgctg ggacttgcac cctgtcagat tcagggagga    15180
   taagttacac tgtggagttt agtttgccta cgcatcatac tgtgcgcctg atccgcgtca    15240
   cagcatcacc ctcagcatga tgggctggca ttcttgaggc atctcagtgt ttgaattgga    15300
   agaatgtgtg gtgaatggca ctgattgaca ttgtgcctct aagtcaccta ttcaattagg    15360
   gcgaccgtgt gggggtgaga tttaattggc gagaaccatg cggccgaaat t             15411

## Description

### FIELD OF THE INVENTION

The present invention relates to an attenuated strain of the Porcine Reproductive and Respiratory Syndrome Virus (PRRSV) and the use thereof as a vaccine in order to induce an immune response.

### BACKGROUND ART

Porcine Reproductive and Respiratory Syndrome virus, commonly indicated with the acronym "PRRS", was isolated for the first time in the Netherlands in 1990 during an outbreak of infection of a reproductive pathology in pig farms (19). Gradually this clinical manifestation extended to other countries, not only in Europe, but also in the United States and Asia (2, 6, 9, 15). The virus was classified in the order *Nidovirales,* family *Arteriviridae,* genus Arterivirus (5, 12). The aforesaid etiological agent is distinguished by an RNA nucleic acid (SS+), and the genome consists of 9 Open Reading Frames (ORFs) coding for different viral proteins, both structural and non-structural (1, 3, 18). Though it was first isolated over 20 years ago, it still represents one of the main problems of a health-related type in pig farms. In addition to pathologies of the reproductive system, it is responsible for respiratory tract infections, often aggravated by secondary infections sustained by other etiological agents with consequent heavy losses of animals and enormous financial damages. The difficulties of controlling this infection are ascribable to two main factors: 1) the continual genetic and antigenic variability of the virus (9, 10, 11, 13, 17); and 2) the inability of the host organism to activate a complete immune response. (14)

The PRRS virus was shown not to be able to activate the passage from the innate response to the adaptive one, with a consequent delay in the synthesis of antibodies neutralizing the viral infectivity and of cytokines such as gamma interferon, resulting in a persistence of the virus in an unopposed form in the organism and its elimination from infected animals after prolonged periods of time and consequent transmission of the infection to other receptive animals. All this leads to the persistence of infection in infected farms.

There exist several vaccines specific for the PRRS virus, attributable to the following types: recombinant, live and attenuated and inactivated (16).

International patent application WO2012153160 relates to a live or inactivated recombinant vaccine, comprising a viral and a pharmaceutically acceptable vehicle, adjuvant and/or excipient, characterised in that the viral vector is a virus capable of generating an immune response due to an increased alpha and/or gamma interferon production, and is capable of quickly replicating, and wherein a nucleotide sequence ORF 5 and ORF 6 of the PRSS virus is inserted in it.

International patent application WO2013017570 relates to the field of attenuated live viruses used as vaccines or medicaments for preventing or treating swine with Porcine Reproductive and Respiratory Syndrome (PRRS), and is based on the discovery of a strain of PRRS virus which is able to induce a type I interferon response in the natural host infected by said etiological agent. In one embodiment, the PRRS virus is a PRRS virus mutant comprising, in comparison with the genome of a wild type strain, a mutation in the gene encoding the not structural protein 1 (nsp1) of said virus.

International patent application WO2013017568 refers to a nucleic acid sequence which comprises the genome of an infectious genotype I (EU) PRRS virus clone useful for studying PRRS and in the development of vaccines, therapeutics and diagnostics for prophylaxis, treatment and diagnosis of PRRS. The patent application discloses that the generation of the infectious clone was started from a virus strain (EUX) recovered from the lungs of a piglet. Patent application US2012189655 relates to an attenuated live vaccine that offers a significant immunological protection to pigs against PRRS.

International patent application WO9800165 relates to vaccines against PRRS produced by attenuation of wild type strains of the virus selected from the group of attenuated NADC-8, NADC-9 and NVSL-14. NADC-8 strain was isolated from the only live pig of a litter that otherwise was presumed to have died as a result of natural infection with PRRSV. Strain NADC-9 was isolated from the lung of a pig that displayed lesions of pulmonary consolidation, presumably as a result of natural infection with PRRS. Strain NVSL-14 was selected from a group of 20 field strains of PRRSV because it replicated to high infectivity titer in a susceptible cell line (MARC-145). Each of the strains was purified by 3 terminal dilutions and then attenuated by a unique process of rapid serial passage (at daily intervals) in MARC-145 cells with dilution of inoculum (≤1:50,000) at each passage, and then tested for attenuation after its 84^{th} and after its 250^{th} passage in cell culture by administration to susceptible pregnant gilts late in gestation.

J. Hu et al., Transboundary and emerging diseases, vol.61, no.2, 2013 discloses the development of live attenuated PRRSV vaccine candidates by multiple serial passages in a continuous cell line, referring to Tian et al. (2009), or by genetically engineering wild-type strains.

The limits of the presently available vaccines are tied to their inability to impart a protective immune response also against viral strains with significant differences in the genomic regions mainly coding for structural glycoprotein 5 (GP5), encoded by ORF 5 and, to a lesser extent, the internal nucleocapsid protein (N), encoded by ORF 7. Moreover, subunit vaccines or ones that use recombinant vectors are scarcely effective as they do not evoke the organism's response in a complete manner.

### DESCRIPTION OF THE INVENTION

The inventors selected a strain of the Porcine Reproductive and Respiratory Syndrome Virus (PRRSV) that is characterised by a high attenuation of virulence and which is capable of producing an antibody response typical of the PRRS virus.

Inventors have in fact surprisingly found out that by conducting serial PRRS infections *in vivo* in the natural host, i.e. pig, the hosts showed a large reduction in the virulence of the PRRS virus. Indeed, the infected pigs presented less severe clinical symptoms while the viremia was still detectable at a lower degree and for a reduced time.

Inventors analysed the characteristic of the inoculated virus at each infection cycle according to the following parameters:
- clinical manifestations and hyperthermia
- presence of the virus at hematic level (viremia)
- stimulation of the immune system (humoral response)
- macroscopic observations carried out at necropsy level
- presence of the virus in the target organs.

Firstly, the virus was able to induce severe clinical manifestations in the infected animals which presented symptoms characterized by lethargy, tachypnea, tremors, eyelid oedema, cough, dyspnoea and persistent hyperplasia of the lymph nodes in the groin up to 20 days. Some subjects also died. The animals also presented hyperthermia with values higher than 40°C for days.

The virus was detectable in the blood serum from the second day of infection and persisted for more than 20 days, in some cases until the 30^{th} days,

The infection activated a classic antibody response already detectable after 7 days, with higher values at 14^{th} days.

In the necropsi phase animals presented interstitial pneumonia, splenomegaly and hyperplasia of the lymph nodes in the groin. The virus was present in the lung, tonsils and in the mediastinal and inguinal lymph nodes.

Said conditions gradually modified in the following passages and the infected pigs presented clinical symptoms of minor entity, while viremia was still detectable.

From the 18^{th} passage *in vivo,* the clinical manifestations further gradually reduced to lead also to the absence of hyperthermia.

The inventors then repeated the passages *in vivo* in order to obtain in the pigs, for at least 4 subsequent passages, absence of clinical symptoms, moderate hyperthermia (body temperature not higher than 39°C), absence of viremic phase, of histopathologic lesions in the target organs (lungs, tonsils, mediastinal lymph nodes) and of interstitial pneumonia and hyperplasia of the lymph nodes. Inventors found out that the virus was still present in the lungs and tonsils and sporadically in the lymph nodes and that it maintained the ability to stimulate the immune system with induction of humoral antibodies. Inventors then isolated the viral strain, adapted the selected and purified virus strain to cell cultures, and sequenced its genome.

It is herein disclosed an attenuated strain of the Porcine Reproductive and Respiratory Syndrome (PRRS) virus characterized by comprising a polynucleotide coding for at least one protein comprising an amino acidic sequence selected from the group consisting of: SEQ ID NO: 2, SEQ ID NO: 3, SEQ ID NO: 4, SEQ ID: 5, SEQ ID NO: 6, SEQ ID NO: 7, SEQ ID NO: 8, SEQ ID NO: 9 and SEQ ID NO: 10 or functional fragments or functional derivatives thereof.

Preferably, said polynucleotide codes for at least one protein consisting of an amino acidic sequence selected from the group consisting of: SEQ ID NO: 2, SEQ ID NO: 3, SEQ ID NO: 4, SEQ ID: 5, SEQ ID NO: 6, SEQ ID NO: 7, SEQ ID NO: 8, SEQ ID NO: 9 and SEQ ID NO: 10. Said polynucleotide preferably comprises an RNA molecule corresponding to at least one polynucleotide comprising a sequence having at least 85 % identity to nt. 218-7327 of SEQ ID NO: 1, or a sequence having at least 89 % identity to nt. 7309-11691 of SEQ ID NO: 1, or a sequence having at least 87 % identity to 11711-12460 of SEQ ID NO: 1, or a sequence having at least 85 % identity to nt. 11716-11928 of SEQ ID NO: 1, or a sequence having at least 89 % identity to nt. 12319-13086 of SEQ ID NO: 1, or a sequence having at least 87 % identity to nt. 12861-13412 of SEQ ID NO: 1, or a sequence having at least 86 % identity to nt. 13409-14014 of SEQ ID NO: 1, or a sequence having at least 90 % identity to nt. 14002-14523 of SEQ ID NO: 1, or a sequence having at least 94 % identity to nt. 14513-14899 of SEQ ID NO: 1. Preferably, the polynucleotide coding for at least one protein as above defined comprises an RNA molecule corresponding to at least one polynucleotide having essentially the sequence nt. 218-7327 of SEQ ID NO: 1, or the sequence nt. 7309-11691 of SEQ ID NO: 1, or the sequence nt. 11711-12460 of SEQ ID NO: 1, or the sequence nt. 11716-11928 of SEQ ID NO: 1, or the sequence nt. 12319-13086 of SEQ ID NO: 1, or the sequence nt. 12861-13412 of SEQ ID NO: 1, or the sequence nt. 13409-14014 of SEQ ID NO: 1, or the sequence nt. 14002-14523 of SEQ ID NO: 1, or the sequence nt. 14513-14899 of SEQ ID NO: 1.

Preferably, the polynucleotide coding for at least one protein as above defined comprises an RNA molecule corresponding to a polynucleotide comprising a sequence having at least 87 % identity to SEQ ID NO: 1.

Preferably, said RNA molecule corresponds to a polynucleotide having essentially the sequence of SEQ ID NO: 1.

Said polynucleotide is preferably cDNA.

In other words, the attenuated strain herein disclosed preferably comprises an RNA molecule coding for at least one protein or functional fragments or functional derivatives thereof as above defined. Preferably, the attenuated strain of the invention comprises an RNA molecule corresponding to a DNA polynucleotide of SEQ ID NO:1.

It is disclosed an attenuated strain of PRRSV comprising an RNA molecule corresponding to a DNA polynucleotide comprising a sequence having at least 87 % identity to SEQ ID NO:1.

It is also herein disclosed an attenuated strain of PRRSV comprising an RNA molecule corresponding to a DNA polynucleotide of SEQ ID NO:1.

In the present strain, the sequence nt. 218-7327 of SEQ ID NO: 1 corresponds to ORF1a, the sequence nt. 7309-11691 of SEQ ID NO:1 corresponds to ORF1b, the sequence nt. 11711-12460 of SEQ ID NO: 1 corresponds to ORF2a, the sequence nt. 11716-11928 of SEQ ID NO: 1 corresponds to ORF 2b, the sequence nt. 12319-13086 of SEQ ID NO: 1 corresponds to ORF 3, the sequence nt. 12861-13412 of SEQ ID NO: 1 corresponds to ORF 4, the sequence nt. 13409-14014 of SEQ ID NO: 1 corresponds to ORF 5, the sequence nt. 14002-14523 of SEQ ID NO: 1 corresponds to ORF 6, and the sequence nt. 14513-14899 of SEQ ID NO: 1 corresponds to ORF 7.

The sequences nt. 1-217 and nt. 14900-15012 of SEQ ID NO: 1 correspond respectively to 5'UTR and 3'UTR of the viral genome.

Preferably, the strain according to the invention was deposited under the Budapest Treaty at the Collection Nationale de Cultures de Micro-organismes (CNCM), with number CNCM 1-4859 on June 5 2014 (deposit information:
Microorganism Deposit Accession No.: CNCM 1-4859;
Taxonomic name: porcine reproductive and respiratory syndrome virus;
Depositary Institution name: Collection Nationale de Cultures de Micro-organismes (CNCM);
Depositary Institution address: Institut Pasteur, 25-28, rue du Docteur Roux, 75724 Paris Cedex 15, France.
Deposit Date: June 5, 2014).

It is therefore an object of the invention an attenuated strain of PRRSV with the above deposit information.

The strain of the present invention was designated as PRRS/IZSLER/2014 by the inventors. The strain according to the present invention is characterised by a high attenuation of virulence and is capable of producing an antibody response typical of the PRRS virus.

It is herein disclosed a protein comprising the amino acid sequence selected from the group consisting of: SEQ ID NO: 2, SEQ ID NO: 3, SEQ ID NO: 4, SEQ ID: 5, SEQ ID NO: 6, SEQ ID NO: 7, SEQ ID NO: 8, SEQ ID NO: 9, SEQ ID NO: 10 or functional fragments or functional derivatives thereof, preferably a protein having essentially the amino acid sequence selected from the group consisting of: SEQ ID NO: 2, SEQ ID NO: 3, SEQ ID NO: 4, SEQ ID: 5, SEQ ID NO: 6, SEQ ID NO: 7, SEQ ID NO: 8, SEQ ID NO: 9, SEQ ID NO: 10.

Herein disclosed are an isolated polynucleotide comprising at least one of the polynucleotides as defined above, or of complementary sequence. An object of the invention is an isolated polynucleotide comprising the RNA molecule corresponding to a polynucleotide having the sequence of SEQ ID NO: 1 or the polynucleotide of SEQ ID NO:1, or the complementary sequence thereof.

Other objects of the invention are a recombinant expression vector comprising the polynucleotide comprising the RNA molecule corresponding to a polynucleotide having the sequence of SEQ ID NO: 1 or the polynucleotide of SEQ ID NO:1, or the complementary sequence thereof.

; a host cell comprising said recombinant expression vector or comprising the polynucleotide as defined above, preferably said host cell being selected from an animal, plant, yeast, bacterium or insect cell, more preferably being a swine or pig cell.

The polynucleotide according to the invention preferably comprises an RNA molecule corresponding to the polynucleotide of SEQ ID NO: 1 or the polynucleotide of SEQ ID NO: 1 or the complementary sequence thereof.

Another object of the invention is an immunogenic composition comprising the strain as defined above , or at least one polynucleotide as defined above, or at least one vector as defined above or at least one host cell as above defined and at least one carrier and / or adjuvant acceptable for veterinary use. The immunogenic composition is preferably for medical use, more preferably for use as a vaccine.

A further object of the invention is the strain as defined above or the polynucleotide as defined above or the vector as defined above, or the host cell as defined above for use as a medicament, in particular for use in vaccine therapy for porcine reproductive and respiratory syndrome. The subject of the therapy is preferably swine or pig.

A further object of the invention is the strain according to the invention or the immunogenic composition of the invention for use in a method for preventing a pig from infection from a PRRSV or developing PRRS.

It is herein disclosed a method for preventing a pig from infecting from a PRRSV or developing PRRS, comprising inoculating the pig with the strain as above defined or with the immunogenic or vaccine composition as above defined.

The immunogenic or vaccine composition as defined above comprises an effective amount of viral particles per dose; it preferably comprises about 10⁵ TCID₅₀/ml where TCID₅₀ is the median infectious dose for tissue cultures ("Tissue Culture Infectious Dose₅₀"). The dose amount of a plasmid as above defined or of a polynucleotide as above defined in a vaccine of the present invention preferably ranges from about 0.1µg to about 100mg. A suitable dosage size ranges from about 0.5ml to about 10ml, and more preferably from about 1ml to about 5ml. In the context of the present invention an "immunogenic or vaccine composition" refers to a composition that will elicit a protective immune response, that may comprise induction of antibodies and/or of a T-cell response, in an animal which was exposed to the vaccine composition.

In the context of the present invention, "polynucleotide" means a polynucleotide of DNA, cDNA or RNA and includes both single-stranded and double-stranded molecules.

Any combination of the above described polynucleotides or proteins may be comprised in the present invention.

In the present invention, "RNA molecule corresponding to at least one polynucleotide" means an RNA molecule that is identical to said polynucleotide, except for the fact that the RNA sequence contains uracil instead of thymine and the backbone of the RNA molecule contains ribose instead of deoxyribose.

For example, the SEQ ID NO:1 is a cDNA sequence corresponding to or encoding the RNA genome of the viral strain of the present invention. Therefore, a DNA sequence complementary to the DNA sequence of SEQ ID NO:1 is for example a template for, i.e. is complementary to or encodes, the RNA genome of the viral strain of the present invention.

In the context of the present invention, the reference to SEQ ID NO:1 includes the SEQ ID NO:1, the RNA sequence corresponding to SEQ ID NO:1 and a DNA or RNA sequence complementary to SEQ ID NO:1.

The term "complementary" sequence refers to a polynucleotide which is non-identical to the sequence but either has a complementary base sequence to the first sequence or encodes the same amino acid sequence as the first sequence. A complementary sequence may include DNA and RNA polynucleotides. The term "functional fragment" or "functional derivative" may be understood as capable of being recognized as an epitope by antibody molecule or as capable of elicit a protective immune response. "Fragments" are preferably long at least 10 aa., 20 aa., 30 aa., 40 aa., 50 aa., 60 aa., 70 aa., 80 aa., 90 aa., 100 aa., 150 aa., 200 aa., 300 aa., 400 aa., 500 aa., 600 aa., 700 aa., 800 aa., 900 aa., 1000 aa., 1200 aa., 1400 aa., 1600 aa., 1800 aa. or 2000 aa. "Derivatives" may be recombinant or synthetic. The term "derivative" as used herein in relation to a protein means a chemically modified protein or an analogue thereof, wherein at least one substituent is not present in the unmodified protein or an analogue thereof, i.e. a protein which has been covalently modified. Typical modifications are amides, carbohydrates, alkyl groups, acyl groups, esters and the like. As used herein, the term "derivatives" also refers to longer or shorter proteins and/or having e.g. a percentage of identity of at least 84%, 85%, 86%, 87%, 88%, 89%, 90%, 91%, 92%, 93%, 94%, 95%, 96%, 97%, 98%, more preferably of at least 99% with SEQ ID NO: 2, SEQ ID NO:3, SEQ ID NO:4, SEQ ID NO:5, SEQ ID NO:6, SEQ ID NO:7, SEQ ID NO:8, SEQ ID NO:9 or SEQ ID NO:10.

In the context of the present invention, "attenuated" strain means a viral strain that is capable of infecting and/or replicating in a susceptible host, but is not pathogenic or is less pathogenic for the susceptible host. For example, the attenuated virus can cause unobservable/undetectable manifestations or milder clinical manifestations, or exhibit a reduction in the efficiency of viral replication and/or infectivity compared to the related isolated field strains. The expression "susceptible" host as used herein refers to an animal that can be infected by PRRSV. When introduced into a susceptible animal, an attenuated strain of PRRSV can also induce an immune response against PRRSV, and in this way make the animal immune to PRRSV infection.

In the present invention "at least 87 % identity" means that the identity may be at least 87 % or 90% or 95% or 100% sequence identity to referred sequences. This applies to all the mentioned % of identity. Preferably, the % of identity relates to the full length of the referred sequence.

In the context of the present invention a carrier may be any vehicle or composition involved in delivery of vaccine into the subject or that facilitated the storage of the composition.

In the context of the present invention, a "vector" may refer to a carrier that can deliver a target polynucleotide into a host cell, as e.g. plasmids and viral vectors.

In the context of the present invention, the "host cell" as above defined is preferably a cell into which the vector can be introduced, for example by transduction, transfection, electroporation, infection or cell fusion.

In the immunogenic or vaccine composition as above defined, suitable carriers may include water, buffers, saline, liposomes, polymeric materials, preservatives, oils, emulsions, stabilizers and/or carrier particles. The skilled man may select suitable carries. A reference for the vaccine formulations is the book by Remington ("Remington: The Science and Practice of Pharmacy", Lippincott Williams & Wilkins, 2000). Regarding the vaccine administration this can be carried out intramuscularly, intranasally, subcutaneously, by aspersion, spraying, or in drinking water.

### SEQUENCES

The present invention will be described through non-limiting examples, making reference to the following figures:

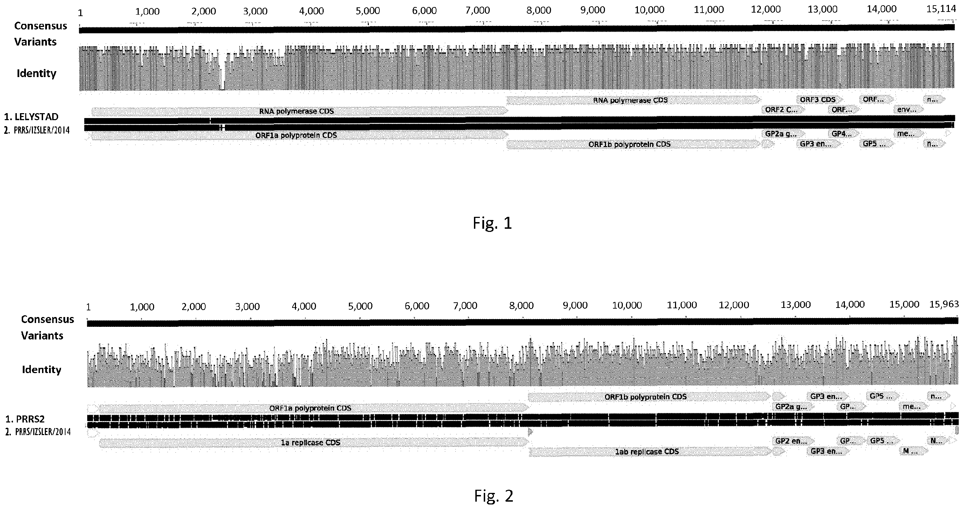

Figure 1. Graphic representation of the degree of identity of the sequences of the virus 14D 4A 3A with the European prototype represented by the Lelystad virus (GenBank M96262.2 (SEQ ID NO:20)). In the upper part of the figure it is reported the extension of the consensus sequence, in the lower part it is reported the alignment of the sequence of the clone 14D 4A 3A with that of the Lelystad virus, and in the centre it is reported the histogram representing the degree of identity between the two sequences along the various portions thereof.
Figure 2. Graphic representation of the degree of identity of the sequences of the virus 14D 4A 3A with the North American prototype VR 2332 (GenBank U87392.3 (SEQ ID NO:21)). In the upper part of the figure it is shown the extension of the consensus sequence, in the lower part it is shown the alignment of the sequence of the clone 14D 4A 3A with that of the North American prototype VR 2332, and in the centre it is shown the histogram representing the degree of identity between the two sequences along the various portions thereof.

### EXAMPLES

The viral strain object of the present invention was selected starting from an isolate (BS/114S/2000) from the blood serum of a pig aborted during an outbreak of PRRS infection in an Italian pig farm. Said isolate was characterised by high virulence not only under field conditions but also under experimental conditions. The animals experimentally infected presented typical symptoms of the pathology, with hyperthermia, depression and anorexia so severe as to even lead to death. The virus persisted in the bloodstream for a prolonged period of time, generally equal to 28 days. (4, 7, 8)

The viral isolate was used to infect 20-day-old Specific Pathogen Free (SPF) pigs originating from the SPF herd of the Istituto Zooprofilattico Sperimentale della Lombardia e dell'Emilia Romagna (Experimental Zooprophylactic Institute of Lombardy and Emilia Romagna), Brescia. The animals were infected intranasally (1 ml/nostril) and intramuscularly (1 ml) and the infected pigs underwent clinical observation and determinations of viremia and antibody response. The animals were sacrificed at the height of the peak of an overt clinical disease and the target organs, represented by the lungs, tonsils and lymph nodes, were individually homogenized, tested for the presence of the PRRS virus and used for further *in vivo* investigations.

The process was carried out for a total of 22 times, using for each new infection the virus obtained from one of the aforesaid organs which displayed the highest viral concentration. The animals were infected intranasally (1 ml/nostril) and intramuscularly (1 ml).

The inventors then repeated the passages *in vivo* in order to obtain in the pigs, for at least 4 subsequent passages. Inventors found out that the virus did not induce clinical signs and hyperthermia was detected for few days and in only few pigs, viremia was detected in some of the infected pigs for short period of time. At necropsy it was shown that virus was still present in the lungs and tonsils and sporadically in the lymph nodes and that it maintained the ability to stimulate the immune system with induction of humoral antibodies.

For the last experiments carried out with virus at the 22nd serial passage on pigs, use was made of four 30-day-old pigs free of PRRS infection and experimentally infected with a tissue homogenate derived from the previous trial. The animals were infected intranasally (1 ml/nostril) and intramuscularly (1 ml) and underwent clinical observations and virologic and serological investigations. The results confirmed the absence of any evident clinical symptoms, sporadic hyperthermia (2 out of 4 subjects) limited to 2-4 days after infection, presence of the virus in the target organs (inguinal lymph nodes, lungs, tonsils) removed during necropsy examination and a serological response. No macroscopic lesions ascribable to infection by the PRRS virus were detectable during necropsy examination. The virus was present in the blood serum samples of only few animals and for brief periods, no more than 4 days, in contrast with what was observed in the first experiments, in which the virus could be found in the bloodstream of all infected animals for up to 28 days.

Virus nucleic acid was evaluated by molecular biology assay. Total RNA was extracted from 200 µl of whole blood or serum using RNeasy™ Mini Kit (Qiagen, Milan, Italy) by QIAcube platform (Qiagen, Milan, Italy) according to the instructions of the manufacturer. Synthesis of the first strand cDNA was carried out in a final volume of 20 µl. Three µl of extracted total RNA was used as template for cDNA synthesis in presence of M-MLV reverse transcriptase and Random Hexamer using GeneAMP kit (AB, Monza, Italy) according to the manufacturer's instructions. The TaqMan® probe Real-Time PCR amplification was performed in the CFX96™ Real-Time System (Bio-Rad, Milan, Italy). The PRRS amplification was performed by One-Step Reaction using primers set and probe designed on ORF 6 gene of PRRS virus. Each 20 µl PCR mixture consisted of 1× QuantiTect Virus Master Mix, 1× QuantiTect Virus RT Mix (Qiagen, Milan, Italy), 0,4 µM of each primer, 0,2 µM of probe and 3 µl of total RNA. The amplifications conditions were: RNA retrotrascription at 50°C for 20 min then 95°C for 5 min followed by 40 cycles of denaturation at 95°C for 15 s, annealing-extension at 60°C for 45 s and plate read (acquisition of fluorescent data). The fluorescence threshold limit of the CFX96™ Real-Time System was set automatically.

Despite this attenuated clinical behavior, the pigs exhibited a typical antibody response of the PRRS virus characterised by the synthesis of non-neutralizing humoral antibodies of the IgG class approximately 7-10 days after infection and a late appearance, approximately 60 days after infection, of neutralizing antibodies.

In particular serological test was carried out by the enzyme linked immunoassay ELISA test by using a commercial kit (IDEXX PRRS X3 Ab Test, USA) .

The PRRS viral strains isolated from pig lungs, tonsils and lymph nodes in said last experiments, in particular the virus found in the lungs of one of these pigs (no. 247), were individually subjected to adaptation and serial cultivation in the MARC-145 cell line (fetal monkey kidney; IZSBS catalogue code: BS CL 127).

Adaptation and culturing in the MARC-145 cell line were obtained only after solving several problems tied to the culture conditions and conceiving a new co-culture strategy, which enabled infected MARC-145 cell lines to be obtained. Previously, the adaptation of the virus BS/114S/2000 to the MARC-145 cell line was achieved by carrying out serial passages and it was not necessary to rely on more sophisticated techniques such as co-culture. This strategy comprises a first step of infection with the PRRS viral strains isolated in the above-mentioned last passage of primary cell cultures of swine alveolar macrophages (SAM), known as being the cells most receptive to the first viral isolation. Following their positivity, the MAS cells infected were jointly cultivated (co-culture method) with cells of the MARC-145 line. The virus was thus capable of replicating in microphages and, once released by the latter, it was absorbed by the MARC-145 cells. This strategy enabled the infection to be transferred to the MARC-145 cell line, in which the virus began to actively replicate, inducing the cytopathic effect (CPE) typical of the PRRS virus.

The virus adapted to the indicated cell line and capable of actively replicating, thus inducing a cytopathic effect (CPE), was used for *in vitro* investigations of a serological and virologic tests. An initial batch of virus with a known infecting concentration (10^{6.74} DCP₅₀ / ml) was then prepared at the 10th passage on the MARC-145 cell line and purified by isolation from 5 plaques and subsequent amplification. For this investigation, the MARC-145 cell line was prepared in 6-well plates with a culture medium enriched with 5% foetal bovine serum and incubated at 37 °C in 5% CO2. Once confluence was reached, the medium was eliminated and the cultures were infected with 10-fold serial dilutions of the virus. After an absorption period of 60 minutes at 37 °C in 5% CO2 it was eliminated and a 1% layer of agar was added to the cultures and left to solidify at room temperature. The plates were then incubated at 37 °C in 5% CO2 and checked daily for the appearance of cellular alterations induced by the virus, which, due to the presence of agar, resulted in plaques. At the highest dilution, some plaques were removed and gently dissolved in culture medium and inoculated in the MARC-145 cell line prepared in 25 cm² bottles. Upon the appearance of CPEs and the destruction of the cellular substrate, the virus was subjected to a further purification process as indicated. The purification process described was carried out three times and three plaques were isolated, indicated with the following codes: 14D2A1A; 14D4A3A; 16D5A2A.

The clone obtained through purification by means of plaques and indicated with the code 14D4A3A was subjected to whole genome sequencing.

### Qualitative tests

All the clones were subjected to investigations aimed at detecting any bacterial, fungal, mycoplasma and viral contaminations.

In particular, they were inoculated in microbiological media specific for the culture of microbial and fungal agents.

Bacteria, fungi and yeast contaminations were investigated following inoculation of 2 ml of each cell suspension in microbiological media (Agar Sabouraud, Triptic Soy Agar and/or Brain Heart Infusion, incubated at 30°C and 37°C, respectively). Moreover, possible mycoplasma contamination was searched for by real-time PCR investigation using the Mycosensor kit (Agilent Company, IT; M-Medical S.r.l., Milano, Italy). Lastly, any viral contaminations were searched for by means of molecular biology reactions specific for the following viruses.
- Pseudorabies (PRV)
- Circovirus
- Torque Teno Virus (TTV)
- Pestivirus (bovine viral diarrhea virus - BVD and classical swine fever - CSF)
- Parvovirus (PPV)
- Porcine lymphotropic herpes viruses (PLHV)
- Porcine endogenous retroviruses (PERVs)
- Influenza A
- Hepatitis E virus (HEV)

### Quality system

The activity was carried out in accordance with the two quality systems applied by the laboratory and represented respectively by:
- ISO 9001 2008 (cell culture production and IZSLER biobank);
- ISO IEC 17025 (reference viruses and methods of analysis).

### Preparation of batches

As indicated previously, only one of the three clones obtained with the plaque method and indicated with the code 14D4A3A was amplified, titrated and subjected to sequencing. The infecting concentration of the batch prepared in the 14th passage on the MARC-145 cell line was equal to 10^{5.74} cytopathic doses (CPD)₅₀/ml calculated in accordance with the formula of Reed and Muench.

### Qualitative tests

The tests carried out showed that the selected viral populations were free of bacterial, fungal, yeast and mycoplasma contaminations and free of extraneous viruses. Bacteria, fungi, yeast and mycoplasma contaminations were investigated as described above.

### Further in vivo experimental investigations

A further *in vivo* experimental investigation was conducted in order to check that the purification processes implemented had not induced alterations in the biological characteristics of the virus, with particular reference to the characteristics of attenuation of the virulence.

For this purpose, an *in vivo* test was made on two 30-day-old PRRS infection-free pigs obtained from a PRRS infection-free farm in the province of Brescia and subjected to infection with the clone 14D4A3A.

The methods were the same as those used in the previous tests and based on intranasal inoculation (1 ml/nostril) and intramuscular inoculation at the same dose (1 ml). The animals exhibited no clinical symptoms and absence of hyperthermia. The virus was detectable in the bloodstream up to the 4th day after infection. From the 5th to 6th day it was detectable in extremely reduced concentrations (at the limit of visualization) and completely undetectable in the following days.

Serological investigations showed an antibody response that was already observable 6 days after infection (1 pig) and at 14 days both pigs exhibited specific antibodies in agreement with what was found in the previous experiments.

In particular serological test was carried out as above described.

The necropsy examination carried out 14 days after infection did not reveal any macroscopic lesions ascribable to PRRS virus infection.

The target organs (tonsils, spleen, lobes of the lungs, pulmonary lymph nodes, mesenteric lymph nodes, inguinal lymph nodes) of the two infected pigs resulted positive and the highest viral concentration was found in the tonsils and lung lobes and apical, as indicated in the following table.

Virus nucleic acid was evaluated by molecular biology assay, according to the methods above described.

The microbiological tests, according to the methods above described, performed on the target organs of the two pigs did not reveal any bacterial or mycoplasma infections.

The viral clone 14D4A3A advantageously isolated did not exhibit any variations in the characteristics of attenuation of virulence. It is indicated by the inventors as PRRS/IZSLER/2014.

**Table 1. Results of real-time PCR reaction carried out on the organs of 2 pigs infected with PRRS clone 14D4A3A.**

| **PIG No.** | **SAMPLE TYPE** | **RESULT** | **Threshold value (threshold cycle** - **CT)** |
|---|---|---|---|
| 19 | Serum | Negative | |
| | Tonsil | Positive | 26* |
| | Spleen | Positive | 35 |
| | Pulmonary lymph nodes | Positive | 31 |
| | Mesenteric lymph nodes | Positive | 36 |
| | Inguinal lymph nodes | Positive | 33 |
| | Apical lung lobe | Positive | 26* |
| | Medial lobe of the lung | Positive | 31 |
| | Caudal lobe of the lung | Positive | 26* |
| | Azygos lobe of the lung | Positive | 36 |
| 20 | Serum | Negative | |
| | Tonsil | Positive | 28* |
| | Spleen | Positive | 31 |
| | Pulmonary lymph nodes | Positive | 33 |
| | Mesenteric lymph nodes | Positive | 36 |
| | Inguinal lymph nodes | Positive | 35 |
| | Apical lung lobe | Positive | 32 |
| | Medial lobe of the lung | Positive | 35 |
| | Caudal lobe of the lung | Positive | 32 |
| | Azygos lobe of the lung | Positive | 28* |

The threshold values indicated with an asterisk are the ones corresponding to a higher concentration of viral nucleic acid.

### Genome sequencing - Annotations and comparison with the reference Lelystad viruses (GenBankM96262.2) and VR-2332 (GenBank U87392.3)

Clone 14D4A3A was centrifuged at 55.000 g/60 min at 4 °C; the pellet was diluted in 2 ml of phosphate buffered saline (PBS) and subjected to genome sequencing as indicated below. Clone 14D4A3A of the PRRS virus was subjected to total RNA extraction with a specific kit with columns (RNeasy mini kit, QIAGEN, Milan, Italy).

The total extracted RNA was subjected to reverse transcription with the PRRS-RT primer (Table 2) specific for the PRRS virus according to standard procedures.

The cDNA obtained was subjected to amplification with specific primers in order to obtain 4 overlapping amplicons capable of covering the entire PRRS genome from the 5'UTR to 3'UTR regions (Table 2).

The amplicons obtained via PCR were then co-purified with the NucleoSpin Gel and PCR Clean-up kit (MACHEREY-NAGEL GmbH & Co. KG, Germany), quantified by spectrophotometry and the DNA library for sequencing was prepared with the Nextera-XT kit (Illumina, Inc. - Worldwide Headquarters, San Diego, CA 92122 USA).

The sequencing reaction was carried out using Illumina MiSeq instrumentation. The viral genome was assembled de novo with DNASTAR NGen software (DNASTAR, Inc, WI, USA) and analyzed with DNASTAR Seqman Pro. A single contig of 15012 nucleotides was obtained with the assembly of 30712 elementary sequences (reads), with an average coverage of 372 (SEQ ID NO: 1). The sequence obtained was annotated with the aid of the GeneQuest DNASTAR software package.

**Table 2. Sequence of the primers used for reverse transcription and amplification of the genome of clone 14D4A3A of the PRRS virus.**

| PRIMER | SEQUENCE |
|---|---|
| PRRS-RT | |
| TER-FW | TGA TGT GTA GGG TAT TCC CCC (SEQ ID NO: 12) |
| TER-RW | CGT GAC CCA CCG AGT AAC TT (SEQ ID NO: 13) |
| SEC-FW | TCT TGA GCA GCG CCA ACT TT (SEQ ID NO: 14) |
| SEC-RW | TCA TAC CAC TCA GGG TCA T (SEQ ID NO: 15) |
| PRRS 8500FOR | CCA GGC GTT YAT GAA GAA AGC (SEQ ID NO: 16) |
| PRRS 12020 REV | AAC AAG CCT TCA TAG GAC CTT CG (SEQ ID NO: 17) |
| PRRS 11910 FOR | TAC TGT TCG GGT TCA CCG TCG C (SEQ ID NO: 18) |
| PRRS END-RW | AAT TTC GGT CAC ATG GTT C (SEQ ID NO: 19) |

The sequence of the PRRSV clone under examination was aligned with those of the reference PRRSV strain Lelystad virus (GenBank M96262.2), as the prototype of European strains, and VR-2332 (GenBank U87392.3), as the prototype of American strains.

The result of the alignments is shown in aggregate form below (Table 3).

**Table 3. Nucleotide sequence similarity between PRRS virus clone 14D4A3 A and Lelystad virus (GenBank M96262.2) and VR-2332 (GenBank U87392.3).**

| Region | % Nucleotide Identity of PRRS virus clone 14D 4A 3A with the Lelystad virus of (GB M96262.2) | % Nucleotide Identity of PRRS virus clone 14D 4A 3A with the virus VR-2332 (GB U87392.3) |
|---|---|---|
| Entire genome | 86% | 61% |
| ORF1a | 84% (with deletion of 84 nt) | 54% |
| ORFlb | 88% | 63% |
| ORF2a | 86% | 63% |
| ORF2b | 84% | 71% |
| ORF3 | 88% | 65% |
| ORF4 | 86% | 65% |
| ORF5-GP5 | 85% | 60% |
| ORF6 | 89% | 69% |
| ORF7 | 93% | 58% |

The PRRS virus clone 14D 4A 3A was aligned with the Lelystad virus (GenBank M96262.2) (European prototype of the PRRS virus) and with VR-2332 (GenBank U87392.3) (American prototype of the PRRS virus) using the ClustalW program; the analysis showed the percentages of nucleotide identity indicated in table no. 3. Figure 1 and Figure 2 are the graphical representation of the alignment of the PRRS virus clone 14D 4A 3A respectively with the Lelystad virus (GenBank M96262.2) and with VR-2332 (GenBank U87392.3) prepared with Geneious.

### REFERENCES

1) Balasuriya and Snijder (2008). "Arteriviruses". Animal Viruses. Molecular Biology. Caister Academic Press. ISBN 978-1-904455-22-6.
2) Barboza, David. Chinese Pig Virus Causes Concern Around the Globe. The New York Times. August 15, 2007.
3) Benfield D, Collins J, Dee S, Halbur P, Joo H, Lager K, et al. Porcine reproductive and respiratory syndrome. In: Straw BE, D'Allaire S, Mengeling WL, Taylor DJ, editors. Diseases of the swine. 8° ed. Ames, Iowa: Iowa State University Press; 1999. pp. 201-32.
4) Candotti P, Dotti S, Amadori M, Villa R, Lombardi G, RotaNodari S, Ferrari M. Experimental infection of pure bred landrace pigs with porcine respiratory and reproductive virus (PRRSV): clinical and laboratory findings. 20th IPVS Internationl Pig Veterinary Science Congress: Durban, South Africa, 22-26 June 2008: proceedings.
5) Cavenagh D. Nidovirales: a new order comprising Coronaviridae and Arteriviridae. Arch Virol 1997; 142: 629-33.
6) Collins J, Benfield D, Christianson W, Harris L, Hennings J, Shaw D, et al. Isolation of swine infertility and respiratory syndrome virus (isolate ATCC VR-2332) in North America and experimental reproduction of the disease in gnotobiotic pigs. J Vet Diagn Invest 1992; 4: 117-26.
7) Corradi A, Ferrari M, Cantoni Am, Robotti C, Alborali L, of Lecce R, Candotti P, Sandri Gp, Borghetti P .Study on the virulence, cell-mediated immune response and histolesivity of three field PRRSV strains with an ORF 5 genetic variation Vet Res Commun . - Vol. 29 suppl 2 (2005). pp. 241-243.
8) Ferrari M, Tosini A, Corradi A, Borghetti P, Scalvini A, Cantoni Am, Ferrando L, Alborali° L. Experimental infection with a porcine reproductive and respiratory syndrome virus (PRRSV) strain with relevant genomic mutations in the ORF5 region. PRRS PMWS swine influenza : 4th International Symposium on emerging and re-emerging pig disease: Rome, June 29th - July 2nd, 2003 : proceedings
9) Song J, Shen D, Cui J, Zhao B (Oct 2010). "Accelerated evolution of PRRSV during recent outbreaks in China". Virus Genes 41 (2): 241-5. PMID 20652733.
10) Kapur V, Elam MR, Pawlovich TM, Murtaugh MP. Genetic variation in porcine reproductive and respiratory syndrome virus isolates in the midwestern United States. J Gen Virol. 1996 Jun; 77 (Pt 6): 1271-6.
11) Lee, C. and Yoo, D. Cysteine residues of the porcine reproductive and respiratory syndrome virus small envelope protein are non-essential for virus infectivity. J Gen Virol. 2005 Nov; 86 (Pt 11): 3091-6.
12) Meulenberg, J. J.; Hulst, M. M.; de Meijer, E. J.; Moonen, P. L.; den Besten, A.; de Kluyver, E. P.; Wensvoort, G., and Moormann, R. J. Lelystad virus, the causative agent of porcine epidemic abortion and respiratory syndrome (PEARS), is related to LDV and EAV. Virology. 1993 Jan; 192 (1): 62-72.
13) Meng XJ, Paul PS, Halbur PG, Morozov I. Sequence comparison of open reading frames 2 to 5 of low and high virulence United States isolates of porcine reproductive and respiratory syndrome virus. J Gen Virol. 1995 Dec; 76 (Pt 12): 3181-8.
14) Murtaugh M.P, Xiao Z, Zuckermann F. Immunological responses of swine to porcine reproductive and respiratory syndrome virus infection. Viral Immunol. 2002; 15: 533-547.
15) Nelsen C, Murtaugh M.P, Faaberg K. Porcine reproductive and respiratory syndrome virus comparison: divergent evolution on two continents. J Virol 1999; 73: 270-80.
16) Petrini S, Ramadori G., Villa R, Borghetti P, De Angelis E, Cantoni AM, Amici A, and Ferrari. Evaluation of Different DNA Vaccines against Porcine Reproductive and Respiratory Syndrome (PRRS) in Pigs. Vaccines 2013, 1(4), 463- 480; doi: 10.3390/vaccines 1040463.
17) Stadejek T, Stankevicius A, Murtaugh MP, Oleksiewicz MB. Molecular evolution of PRRSV in Europe: Current state of play. Vet Microbiol. 2013; 165 : 21-8.
18) Thiel HJ, Meyers G, Stark R, Tautz N, Rumenapf T, Unger G, Conzelmann KK., Molecular characterization of positive-strand RNA viruses: pestiviruses and the porcine reproductive and respiratory syndrome virus (PRRSV). Arch Virol Suppl. 1993; 7: 41-5.
19) Wensvoort G. Lelystad virus and the porcine epidemic abortion and respiratory syndrome. Vet Res 1993; 24: 117-24.

## Claims

1. Attenuated strain of the Porcine Reproductive and Respiratory Syndrome (PRRS) virus deposited under the Budapest Treaty at the Collection Nationale de Cultures de Micro-organismes (CNCM), with n. CNCM 1-4859 on June 5, 2014.

2. Isolated polynucleotide comprising the RNA molecule corresponding to a polynucleotide having the sequence of SEQ ID NO: 1 or the polynucleotide of SEQ ID NO:1, or the complementary sequence thereof.

3. A recombinant expression vector comprising the polynucleotide according to claim 2.

4. Host cell comprising the recombinant expression vector according to claim 3 or comprising the polynucleotide according to claim 2, preferably said host cell being selected from animal, plant, yeast, bacterium or insect cell, more preferably being a swine or pig cell.

5. Immunogenic composition comprising the strain according to claim 1, or at least one polynucleotide according to claim 2, or at least one vector according to claim 3, or at least one host cell according to claim 4, and at least one carrier and / or adjuvant acceptable for veterinary use.

6. The immunogenic composition according to claim 5 for medical use, preferably for use as a vaccine.

7. The strain according to claim 1, or the polynucleotide according to claim 2, or the vector according to claim 3, or the host cell according to claim 4 for use as a medicament, preferably in vaccine therapy of porcine reproductive and respiratory syndrome.

8. The strain according to claim 1 or the immunogenic composition of claim 5 for use in a method for preventing a pig from infection from a PRRSV or developing PRRS.

## Patentansprüche

1. Attenuierter Stamm des PRRS-Virus (Porcine Reproductive and Respiratory Syndrome), der gemäß dem Budapester Vertrag bei der Collection Nationale de Cultures de Micro-organismes (CNCM) unter der Nummer CNCM I-4859 am 5. Juni 2014 hinterlegt wurde.

2. Isoliertes Polynukleotid, welches das RNA-Molekül umfasst, das einem Polynukleotid mit der Sequenz von SEQ ID NO: 1 entspricht oder das Polynukleotid von SEQ ID NO: 1 oder die komplementäre Sequenz davon.

3. Rekombinanter Expressionsvektor, der das Polynukleotid nach Anspruch 2 umfasst.

4. Wirtszelle, die den rekombinanten Expressionsvektor nach Anspruch 3 umfasst oder das Polynukleotid nach Anspruch 2 umfasst, wobei die Wirtszelle vorzugsweise ausgewählt ist aus einer Tier-, Pflanzen-, Hefe-, Bakterien- oder Insektenzelle, bevorzugter einer Schweine- oder Ferkelzelle.

5. Immunogene Zusammensetzung, die den Stamm nach Anspruch 1 oder mindestens ein Polynukleotid nach Anspruch 2 oder mindestens einen Vektor nach Anspruch 3 oder mindestens eine Wirtszelle nach Anspruch 4 sowie mindestens einen Träger und/oder Hilfsstoff umfasst, der für veterinärmedizinische Zwecke verträglich ist.

6. Immunogene Zusammensetzung nach Anspruch 5 zur medizinischen Verwendung, vorzugsweise zur Verwendung als Impfstoff.

7. Stamm nach Anspruch 1 oder Polynukleotid nach Anspruch 2 oder Vektor nach Anspruch 3 oder Wirtszelle nach Anspruch 4 zur Verwendung als Arzneimittel, vorzugsweise bei der Impfstofftherapie des reproduktiven und respiratorischen Schweine-Syndroms.

8. Stamm nach Anspruch 1 oder immunogene Zusammensetzung nach Anspruch 5 zur Verwendung in einem Verfahren zur Verhinderung der Infektion eines Schweins durch ein PRRSV oder der Entwicklung von PRRS.

## Revendications

1. Souche atténuée du Virus du Syndrome Reproducteur et Respiratoire Porcin (VSRRP) déposée conformément au Traité de Budapest à la Collection Nationale de Cultures des Micro-Organismes (CNCM) sous le numéro CNCM I-4859 le 5 juin 2014.

2. Polynucléotide isolé comprenant la molécule d'ARN correspondant à un polynucléotide ayant la séquence SEQ ID N°1 ou le polynucléotide de SEQ ID N°1, ou la séquence complémentaire de celui-ci.

3. Vecteur d'expression recombinant comprenant le polynucléotide selon la revendication 2.

4. Cellule hôte comprenant le vecteur d'expression recombinant selon la revendication 3, ou comprenant le polynucléotide selon la revendication 2, ladite cellule hôte étant de préférence sélectionnée parmi une cellule d'animal, de plante, de levure, de bactérie ou d'insecte, étant plus préférablement une cellule de porc.

5. Composition immunogène comprenant la souche selon la revendication 1, ou au moins un polynucléotide selon la revendication 2, ou au moins un vecteur selon la revendication 3, ou au moins une cellule hôte selon la revendication 4, et au moins un transporteur et/ou adjuvant acceptable pour un usage vétérinaire.

6. Composition immunogène selon la revendication 5 pour un usage médical, de préférence pour être utilisée comme vaccin.

7. Souche selon la revendication 1, ou polynucléotide selon la revendication 2, ou vecteur selon la revendication 3, ou cellule hôte selon la revendication 4, destiné(e) à être utilisé(e) comme médicament, de préférence comme thérapie de vaccination contre le syndrome reproducteur et respiratoire porcin.

8. Souche selon la revendication 1 ou composition immunogène selon la revendication 5 destinée à être utilisée dans un procédé visant à empêcher un porc d'être infecté par le VSRRP ou de développer le SRRP.
